(19) Europäisches Patentamt European Patent Office Office européen des brevets

(11) **EP 2 068 741 B1**

(12) **EUROPEAN PATENT SPECIFICATION**

(45) Date of publication and mention
of the grant of the patent:
**01.08.2012 Bulletin 2012/31**

(51) Int Cl.:
***A61B 18/18*** (2006.01)

(21) Application number: **07824095.9**

(22) Date of filing: **10.10.2007**

(86) International application number:
**PCT/GB2007/003841**

(87) International publication number:
**WO 2008/044012 (17.04.2008 Gazette 2008/16)**

(54) **OESOPHAGEAL TREATMENT APPARATUS**

SPEISERÖHREN-BEHANDLUNGSGERÄT

APPAREIL DE TRAITEMENT OESOPHAGIEN

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **10.10.2006 GB 0620061**

(43) Date of publication of application:
**17.06.2009 Bulletin 2009/25**

(73) Proprietor: **Medical Device Innovations Limited Cheshire WA4 4FS (GB)**

(72) Inventors:
• **HANCOCK, Christopher Paul**
  **Bristol Avon BS5 8QZ (GB)**

• **WHITE, Malcolm**
  **Bath And North East Somerset BA3 5LW (GB)**
• **WALL, Peter**
  **Newport Gwent NP18 1JX (GB)**

(74) Representative: **Johnson, Richard Alan et al**
**Mewburn Ellis LLP**
**33 Gutter Lane**
**London**
**EC2V 8AS (GB)**

(56) References cited:
| | |
|---|---|
| WO-A-2005/112812 | US-A- 6 024 743 |
| US-A1- 2003 009 165 | US-A1- 2003 069 620 |
| US-A1- 2004 002 747 | US-A1- 2005 096 647 |

**Description**

TECHNICAL FIELD

This invention relates to apparatus for treating tissue using thermal energy derived from microwave radiation. For example, it relates to a technique of causing tissue necrosis (thermal damage) and/or tissue ablation by exposing tissue to radiation with a frequency in the range of 5 to 60 GHz.

The present invention is particularly aimed at treatment of the oesophagus (also known as the esophagus or gullet), e.g. to treat the conditions known as gastro-oesophageal reflux and Barrett's oesophagus. Although the present application is framed around this use, it may also provide a means of treating other medical conditions that are accessible by insertion of a treatment instrument through a natural orifice, (e.g. mouth, nose, anus or urethra). It may also be applicable to treatment in open surgical procedures.

BACKGROUND TO THE INVENTION

The oesophagus is the muscular tube that carries food from the mouth to the stomach and is lined by cells similar to those that form the skin (squamous cells).

Barrett's oesophagus is a condition first identified by a surgeon called Norman Barrett in the early 1950's where abnormal cells develop on the inner lining of the lower part of the gullet (oesophagus). The lining at the lower end of the gullet is found to change from squamous to being like the lining of the stomach, or gastric or intestinal metaplasia).

Barrett's oesophagus is a pre-cancerous condition. Over a variable period of time it can occasionally lead to cancer developing in the lower part of the oesophagus. A cancer happens when cells in the affected area become increasingly abnormal (dysplastic) and may eventually become malignant.

The main cause of Barrett's oesophagus is gastro-oesophageal reflux i.e. juices from the stomach 'splashing' up into the oesophagus. Gastric juice contains acid, bile and proteins, which help to digest food. The stomach is lined by tissue that is resistant to acid, but the oesophagus is not. Normally, a valve at the bottom of the oesophagus, the lower oesophageal sphincter (LOS), prevents acid from splashing up into the gullet. However, some people have a weak valve, which allows the acid to flow backwards into the oesophagus (reflux). The acid may inflame and irritate the oesophagus, and in some people will cause symptoms of pain and heartburn. This is often referred to as reflux oesophagitis.

It is desirable to destroy the abnormal cells. It is necessary for surgery to be carried out to help strengthen the valve at the bottom of the oesophagus to prevent further acid reflux, or to remove the affected area. Controlled thermal ablation may be used to destroy abnormal cells and provide a means of controllably tightening the valve.

Conventional methods used for treating Barrett's oesophagus include: surgery, low frequency electrosurgery, photodynamic therapy, and argon laser coagulation.

Gastro-oesophageal reflux disease, commonly referred to as GERD, or acid reflux, is a condition in which the liquid content of the stomach regurgitates (backs up, or refluxes) into the oesophagus. The liquid can inflame and damage the lining of the oesophagus. The refluxed liquid usually contains acid and pepsin that are produced by the stomach. Pepsin is an enzyme that begins the digestion of proteins in the stomach. The refluxed liquid also may contain bile that has backed-up into the stomach from the duodenum. Acid is believed to be the most injurious component of the refluxed liquid. Pepsin and bile also may injure the oesophagus.

GERD is a chronic condition. Once it begins, it usually is life-long. If there is injury to the lining of the oesophagus (oesophagitis), this also is a chronic condition. Moreover, after the oesophagus has healed with treatment and treatment is stopped, the injury will return in most patients within a few months. Once treatment for GERD is begun, therefore, it usually will need to be continued indefinitely albeit intermittently in some patients.

The reflux of the stomach's liquid contents into the oesophagus occurs in most normal individuals. In patients with GERD, the refluxed liquid contains acid more often, and the acid remains in the oesophagus longer.

As is often the case, the body has mechanisms to protect itself from the harmful effects of reflux and acid. For example, most reflux occurs during the day when individuals are upright. In the upright position, the refluxed liquid is more likely to flow back down into the stomach due to the effect of gravity. In addition, while individuals are awake, they repeatedly swallow, whether or not there is reflux. Each swallow carries any refluxed liquid back into the stomach. Finally, the salivary glands in the mouth produce saliva, which contains bicarbonate. With each swallow, bicarbonate-containing saliva travels down the oesophagus. The bicarbonate neutralizes the small amount of acid that remains in the oesophagus after gravity and swallowing have removed most of the liquid.

Gravity, swallowing, and saliva are important protective mechanisms for the oesophagus, but they are effective only when individuals are in the upright position. At night while sleeping, gravity is not in effect, swallowing stops, and the secretion of saliva is reduced. Therefore, reflux that occurs at night is more likely to result in acid remaining in the oesophagus longer and causing greater damage to the oesophagus.

In the US alone, 14 million patients suffer from GERD.

Low frequency RF (i.e. radiation with frequencies in the range 100 kHz to 10 MHz) has been used to treat GERD. Treatment probes are inserted via endoscopes through the mouth and a balloon is inflated to open a plurality of wires. The low frequencies limit the control and speed of ablation, and uniformity of controlled thermal damage is difficult to achieve due to the fact that the number of wires are limited to around six. In systems such as these, heat induced collagen contraction is produced.

US 2003/0009165 discloses a GERD treatment apparatus for insertion into the oesophagus. The apparatus has an expandable member for dilating the oesophagus and locating an energy delivery device at a region to be treated, such that electric current can flow through targeted tissue to cause heating that leads to cell necrosis.

US 2004/0002747 discloses a medical device having an expandable tubular structure comprising coaxially arranged electrode needles for placing into tissue to perform thermal treatment of that tissue.

US 6,024,743 discloses an apparatus for treating a uterus, which comprises an expandable member having an energy delivery device for applying electric current through endometrial tissue to cause heating that leads to cell necrosis.

US 2005/0096647 discloses a catheter for remodelling or removing atherosclerotic material from a blood vessel.

WO 2005/112812 discloses apparatus for ablating target tissue in the left atrium of a patient. The apparatus comprises a balloon that is expandable to bring ablating elements, which may be microwave antennae, into contact with the target tissue.

US 2003/0069620 discloses a balloon catheter in which a heating element is covered by the balloon, and heat is conducted to tissue in contact with the expanded balloon through fluid in the balloon and the balloon itself.

SUMMARY OF THE INVENTION

The present invention seeks to ameliorate the problems associated with the treatment of the oesophagus by providing a new treatment apparatus. The present invention proposes the use of microwave radiation to cause controlled thermal damage around the inside of the oesophagus, which may act to prevent acid reflux. Throughout this specification, "microwave" is used to define the frequency range from 5 GHz to 60 GHz. In practice, the frequency range preferred for use in the present invention is between 14 GHz and 15 GHz. In a specific embodiment described below, a single frequency of 14.5 GHz is used.

According to the invention, there is provided probe having an access configuration in which it is insertable into an oesophagus and a treatment configuration in which it is operable to treat an area of oesophageal wall tissue with microwave radiation, the probe having: a flexible substrate; one or more radiating elements on the flexible substrate; a feed structure arranged to energise the radiating element(s), thereby causing the radiating element(s) to emit microwave radiation; and deployment means arranged to transfer the probe from its access configuration to its treatment configuration, wherein the feed structure includes a grounded conductor on the flexible substrate, and the radiating element(s) comprise either: (i) a plurality of conducting patches arranged in conjunction with the grounded conductor to convey alternating current (AC), each conducting patch having a largest length or width dimension that is a half wavelength of the microwave radiation conveyed by the feed structure when loaded by the tissue to be treated; or (ii) a plurality of non-conducting slots formed in one or more conducting strips, each conducting strip being arranged in conjunction with the grounded conductor to convey alternating current (AC), and wherein, in the treatment configuration, the radiating element(s) are arranged to deliver outwardly a substantially uniform radiation field to transfer energy primarily by radiation to the area of oesophageal wall tissue to be treated.

The frequency of microwave radiation used in the invention is preferably chosen to enable the dominant energy transport mechanism between the probe and the oesophageal wall to be radiation (i.e. conduction is preferably minimised) . The depth of penetration of the radiation into the tissue affects the degree of controlled thermal damage. For oesophageal wall treatment, the depth of penetration of tissue necrosis (thermal damage) is preferably less than 5 mm and more preferably between 1 and 2 mm. Physically the oesophagus comprises a superficial outer layer of between 0.5 and 0.8 mm thickness and a muscle layer of about 0.3mm thickness. This will be especially the case where lossy material is present between the radiating surface of the probe and the surface of the treatment tissue. For example the interface may be filled with air, body fluid or saline. In the treatment configuration, it is preferable for the radiating elements to be in contact or close proximity (e.g. 1 mm or less) with the tissue to be treated. A distance of greater than 1 mm may mean that the energy delivered by the radiating elements drops too much to be of use in treatment.

Preferably, the frequency of microwave radiation is chosen to provide the desired penetration depth. For use in the oesophagus, microwave radiation having a frequency of between 14 and 15 GHz is used. More preferably, microwave radiation having a frequency of 14.5 GHz is used. The depth of penetration for microwave radiation having this frequency (defined as the distance into tissue at which the power of the radiation reduced to $1/e$ (about 37%) of the initial delivered power) is 1.7mm for the oesophagus. This value is calculated using models and data given in "An Internet resource for the calculation of the Dielectric Properties of Body Tissues in the frequency range 10 Hz - 100 GHz" IFAC-CNR, Florence, Italy, 1997-2002 (at http://niremf.ifac.cnr.it/tissprop).

A further advantage of using high frequency microwave energy is that, even where conduction is also required to create the desired level of thermal damage, the treatment time can be kept short because delivered power increases with frequency and the probe may be arranged to match the impedance of the source with the impedance of the load (tissue to be treated).

Preferably, the substrate includes a grounded conductor, wherein the feed structure is arranged to provide alternating current (AC) power to the radiating element(s) and grounded conductor. The grounded conductor is preferably separated from the radiating elements e.g. by having a layer of dielectric material in between. The grounded conductor may also be on the same plane as the radiating elements, arranged to provide a coplanar waveguide antenna, whereby the lines of radiation are between the ground conductor and the signal conductor, both of which are on the same surface.

The feed structure may include a co-axial cable arranged to provide the energy to the radiating elements. The energy is preferably provided through the feed structure as alternating current (AC) to the radiating elements and the grounded conductor.

Preferably, the feed structure includes transmission lines for carrying power through the substrate to the radiating elements. Preferably, the transmission lines are shielded to prevent or minimise radiation from coupling into the oesophageal wall. For example, they may lie in a plane located radially inward from the radiating elements to prevent radiation from the transmission lines from coupling into the oesophageal wall. The transmission lines may be separated from the radiating elements by a layer of dielectric material. Electromagnetic coupling through a layer of dielectric material may be provided to link the feed structure to the radiating elements. Thus, the substrate may comprise a layered structure including an inner (lower) signal conductor, e.g. feed lines, a first dielectric layer separating the inner conductor from the grounded conductor, and a second dielectric layer separating the feed lines from an outer (upper) conductor (e.g. patch antennas). The first and second dielectric layer may be formed from the same or different material, e.g. the first dielectric layer may be a low permittivity Duroid material and the second dielectric layer may be a high permittivity laminate. Specific examples of materials include Rogers high frequency circuit materials, e.g. R03003™ ($\varepsilon_r$ = 3) and R03010™ ($\varepsilon_r$ = 10.2).

Preferably, the thickness of the layer between the radiating elements and the feed lines is as thin as possible in order to couple the maximum energy into the radiating antenna patches. It may also be preferable for the relative permittivity of the dielectric material between the radiating patches and the feed lines to be higher than the relative permittivity of the material between the ground plane and the feed lines in order to concentrate the field.

It is preferable for the substrate to exhibit a low loss characteristic at the frequency of interest to ensure that microwave energy is not lost in the substrate material and that the substrate material does not get excessively hot. Substrate loss can be described by the property tanδ, which is the ratio of energy dissipated to the energy stored. For example, a low loss material may exhibit a tanδ of less than 0.0001 at the frequency of interest.

The radiating elements may be formed in or on or attached to the layer of dielectric material in the substrate. Preferably, each radiating element comprises a conducting antenna patch mounted on the layer of dielectric material. Thus, the substrate may include a layer of e.g. low loss flexible microwave dielectric material that separates two conductors, e.g. an inner and outer conductor. The outer conductor may be adapted to fabricate the patch antenna arrays.

Thus, the present invention preferably provides super high frequency (SHF) microwave radiation (commonly defined as the range 3 GHz to 15 GHz) through the feed structure to energise a plurality of radiating patch antennas. Preferably, the adjacent antenna patches are in close proximity to one another to provide a uniform field to facilitate the uniform tissue heating effect around the circumference of the oesophagus. In this regard, the largest dimension (length or width) of each antenna patch is preferably half the loaded wavelength ($\lambda_L$), where the loaded wavelength is a function of the relative permittivity of the substrate material, the relative permittivity of the biological tissue, and the frequency of operation.

Preferably, the centres of adjacent antenna patches are spaced from each other.by one $\lambda_L$. This helps to ensure that the emitted field is substantially uniform between the probe and the surrounding tissue to be treated.

Preferably, the chosen frequency and tissue/dielectric loading enables patch sizes to be small enough to produce uniform tissue ablation or thermal damage. Thus, the frequency of the radiation, and the impedance of the probe (total feed device) may be selected and, where appropriate, adjusted according to the particular circumstances of use so that a uniform field occurs within the penetration depth of the radiation into the tissue. The energy distribution for a given configuration may be modelled using finite element analysis programs, e.g. Computer Simulation Technology (CST), wherein the electric fields are split into a number of finite elements.

The impedance of the feed structure depends on its geometry. For example, if a microstrip line with a feed width w and substrate (dielectric shield) height h and relative permittivity $\varepsilon_r$ is used, the impedance ($Z_0$) for a narrow strip (w/h < 3.3) is given by:

$$Z_0 = \frac{119.9}{\sqrt{2(\varepsilon_r + 1)}} \left( \ln\left( 4\frac{h}{w} + \sqrt{16\left(\frac{h}{w}\right)^2 + 2} \right) \right),$$

and for a wide strip (w/h > 3.3) is given by

$$Z_0 = \frac{\dfrac{119.9\pi}{2\sqrt{\varepsilon_r}}}{\dfrac{w}{2h} + \dfrac{\ln 4}{\pi} + \dfrac{\ln\left(e\pi^2/16\right)}{2\pi}\left(\dfrac{\varepsilon_r - 1}{\varepsilon_r^{\,2}}\right) + \dfrac{\varepsilon_r + 1}{2\pi\varepsilon_r}\left(\ln\left(\dfrac{e\pi}{2}\right) + \ln\left(\dfrac{w}{2h} + 0.94\right)\right)}.$$

On the other hand, if the feed line is a co-axial cable, then the impedance is given by:

$$Z_0 = \frac{138}{\sqrt{\varepsilon_r}}\log_{10}\frac{b}{a},$$

where a is the outer diameter of the inner conductor and b is the inner diameter of the outer conductor.

Preferably, the feed structure is arranged to cause electromagnetic fields emitted by adjacent conducting patches to be orthogonal to one another. Thus, adjacent patches preferably radiate along edges that are orthogonal to each other. This helps to facilitate uniform field effect over the whole treating surface area. The orthogonal electromagnetic fields may be caused by varying the location of the connection point between feed structure and adjacent conducting patches.

In a preferred arrangement, the substrate comprises a flexible sheet. For example, the substrate may include a laminated sheet having upper and lower conducting layers separated by a dielectric layer. The upper layer may be discontinuous, i.e. it may comprise a plurality of discrete conducting patches. Two or more sheets of this type of material (having conducting surfaces on one or both sides of a dielectric sheet) can be combined to form the layered structure described above. Such structures, having dielectric layers formed of liquid crystalline polymer are known, e.g. the "R/ flex" circuit materials manufactured by Rogers Corporation. Flexible patch antenna structures formed on sheets in this way may be rolled into small diameter tubes suitable for insertion through the oesophagus (or other natural orifices). Thus, a sheet having patch antennas formed on one of its surfaces can form a tubular array of those antennas when it is rolled into a tube. It may be preferable to make metallisation non-continuous on both sides, i.e. the ground plane may be discontinuous to allow for enhanced flexibility.

The feed structure may be arranged in a variety of ways to provide power to the radiating elements (e.g. patch antennas). For example, the tubular array may include a plurality of radiating elements connected in series along a transmission line. This type of construction may be called a "leaky feeder", which may at its simplest comprise a shielded transmission line that is exposed, e.g. through slits or the like at various points along its length, so that it radiates at the exposed points. Each exposed point therefore corresponds to a radiating element. It is preferable for each radiating element to radiate only a small fraction of the total power carried by the transmission line and for each element to radiate the same amount of energy to ensure uniform field intensity along the length of the line. The impedance of each radiating element is preferably lower than the characteristic impedance of the transmission line. The size of adjacent elements may be varied progressively down the line to compensate for the loss in power due to radiation from the earlier radiating elements.

Alternatively or additionally, the tubular array includes a plurality of radiating elements connected in parallel by transmission lines. In this case, it is preferable that the impedance of each radiating element is matched to the impedance of the transmission line to which it is connected to ensure that a field having uniform intensity is provided at the radiating elements. The matched condition prevents reflections if the incident energy from occurring. The impedance may be matched by having a quarter wave transformer located at the junction between each radiating element and its transmission line. The quarter wave transformer may comprise a transmission line of predetermined impedance having a length of

$$\frac{(2n-1)\lambda_L}{4},$$

where $\lambda_L$ is the loaded wavelength. The loaded wavelength is typically a function of the operating frequency and the relative permittivity of the substrate material and the relative permittivity the biological tissue (when the radiating elements are in contact with that tissue). This can be used e.g. when all the transmission lines have a common impedance value. Where the impedance of the transmission lines varies, the quarter wave transformer may include a transmission line of length $\lambda_L/4$ whose impedance is selected, e.g. by calculation to effect impedance matching between the structures to be connected. This theory is conventional, e.g. described by Gang et al in Chapter 12 of "Microstrip Antenna Design Handbook" (published in 2001 by Artech House).

The feed structure may comprise a cascading series of circuit junctions, each comprises the split of a single transmission line into two or more branches, and wherein each branch of the cascading series terminates in a radiating element (e.g. patch antenna). Preferably, impedance matching, e.g. by provision of a quarter wave transformer is provided at each circuit junction. For example, if a 50 Ω feed line was to be split into seven parallel conducting patches each with an impedance of 14 Ω, the overall output impedance would be 14/7 = 2 Ω, so a quarter wave transformer would need an impedance of

$$Z_{trans} = \sqrt{(Z_{in}Z_{out})} = \sqrt{(50 \times 2)} = \sqrt{100} = 10 \ \Omega.$$

Preferably, the probe includes at least 5 radiating elements on the substrate. The greater the number of radiating elements, the more likely it is that uniform thermal damage will occur. The probe may therefore provide an active (radiating) area of a suitable (useful) size to radiate a whole treatment region with the uniform radiation simultaneously.

The radiating elements may contact the tissue to be treated. However, since it is preferred for the main energy transfer mechanism to be radiation (i.e. energy transfer due to conduction is desirably minimised). Preferably, the radiating elements have a biocompatible coating. More preferably, both the radiating patch antenna array and the return (inner or lower) conductor (i.e. the grounded conductor) are covered with a biocompatible material. This may be achieved by applying a conformal coating of e.g. Parylene C to the structure. It is preferable to apply a coating of less that 100 $\mu$m, and more preferable to apply a coating thickness of about 10 $\mu$m so that the biocompatible layer is transparent to the microwave energy.

The radiating element(s) may be provided in the form of a coplanar waveguide. Here the grounded conductor and radiating element(s) are on the same side of the substrate. For example, a conducting strip (e.g. made from metal) may be formed on one side of the substrate and connected to the feed structure to receive a drive signal. The conducting strip may be surrounded on both sides by grounded conducting strips which will cause transmission in a quasi-TEM mode.

The advantage of this structure over conventional co-planar structures is that much less field enters the substrate. Also, the open-nature of the structure results in a large amount of radiation being transmitted between the centre strip and the neighbouring ground conductors. Whilst this is usually an undesirable feature for a conventional transmission line where the aim is to transmit as much power as possible from a source to a matched load, this feature is extremely desirable for the present application, whereby it is desirable that as much of the radiation as possible enters the biological tissue. A further advantage of this structure is that the ground plane is not required on the under side of substrate, which means that the structure may be more flexible.

In the coplanar waveguide arrangement described above, the material (substrate or oesophagus) with the highest relative permittivity should dominate, i.e. the highest fields (energy) will be inside the material with the greatest relative permittivity.

The substrate may include a tubular section which is radially expandable from the access configuration to the treatment configuration, the radiating element(s) being on the tubular section such that, in use in the treatment configuration, they are in close proximity or in contact with the area of oesophageal wall tissue to be treated. Preferably, the tubular section is an inflatable surgical balloon. For example, a water filled balloon may be employed where the microwave feed structure is combined with the channel that the water is pumped through. The surgical balloon may be of the conventional type, e.g. made of nylon, PVC, PET or polyurethane. The radiating elements may be formed (e.g. attached or deposited) on the balloon surface. Alternatively or additionally, the balloon surface may be or include a foil layer shaped to create the radiating elements.

Preferably, the balloon is inflatable from an access configuration where its diameter is 12 mm or less (e.g. about 0.5 mm) to a treatment configuration where its diameter is of a size suitable for the hollow tube (e.g. oesophagus) being treated. For example, the balloon may inflate to a diameter of up to 50 mm, preferably up to 20 mm.

Air may be used to inflate the balloon, but water is preferred because less pressure is required for inflation and there is less risk of harm to the patient in the event of the balloon being damaged. The balloon may be filled with water or air using a syringe with a pressure gauge, and this arrangement may be used to inflate and deflate the balloon.

Any of the slotted antenna structure ("leaky feeder"), the coplanar waveguide or the conducting patch antenna structure defined above may be fabricated on to the surface of the balloon to act as the radiating elements. For example, the balloon (e.g. dielectric substrate) may have a conducting (e.g. metal) grounded layer on its inner surface and conducting patches on its outer surface. The conducting patches on the outer surface may be coated with a biocompatible material.

The radiating element(s) may be on the inside surface of the balloon, which obviates the need for an additional biocompatible layer or for a biocompatible conducting material (e.g. medical grade stainless steel).

The balloon may have two layers: an inner dielectric material and an outer metal foil layer. A slotted antenna may be fabricated by removing portions of the outer metal foil layer.

Typically surgical balloons are fed with an inflating medium (e.g. air or water) through a tube. In a development of the present invention, the inventors propose adapting the feed tube so that it may simultaneously perform the function of the feed structure for the radiating elements. For example, the feed tube may be arranged as a co-axial cable by providing conducting layers on its inner surface and its outer surface. Preferably the end of the feed tube is adapted so that the feed structure is impedance matched to the radiating elements. For example, the end of the feed tube can be arranged as a quarter wave transformer (e.g. having a length of

$$\frac{(2n-1)\lambda_L}{4},$$

as explained above.

An impedance matching transformer may be provided at one or both of the proximal end and the distal end of the co-axial feed tube. This may be advantageous due to the fact that a standard water feed pipe may be used with a coating of metallisation on the walls of the tube to form the microwave co-axial cable. It may also be advantageous to choose an impedance that provides the lowest power loss. It may also be possible to have an impedance transformer at the proximal end only and choose a characteristic impedance such that impedance matching at the distal end between the co-axial feed and the radiating probe(s) is not required.

In another arrangement, the feed structure may comprise a semi-rigid cable (feed cable) which terminates at a flexible patch element (hereinafter referred to as a "paddle"). The radiating elements may be fabricated or mounted on the paddle. They may be a "leaky feeder" or an array of conducting patches as described above. Preferably, the paddle is movable relative to the feed cable from an aligned position suitable for insertion e.g. into the oesophagus to a transverse position where the radiating elements are brought to a position suitable for treatment. For example, the paddle may comprise an elongate member that is substantially parallel to the feed cable in the aligned position but is at an angle (e.g. up to 90°) to the feed cable in the transverse position. The deployment means in this case is arranged to transfer the paddle from the aligned position to the transverse position. The deployment means may include a control (mechanical) wire having one end e.g. attached to a distal end of the paddle and its other end protrudable from the oesophagus to be accessible to a user. Nitinol wire may be used to open and close the paddle structure. In a particular embodiment, the radiating paddle may be attached to the nitinol wire and the nitinol wire used as a memory element to remember the shape required inside the body. A plurality of paddles may be used within the radiating balloon structure described above to ensure that each of the radiating elements produce uniform radiation along the length of the element to cause uniform thermal damage.

The radiating elements may comprise one or more monopoles. For example, a monopole may be provided e.g. at the distal end of the paddle described above to provide a user-directable microwave field within the oesophagus. Alternatively, the feed cable may terminate in a plurality of monopoles. Preferably, the plurality of monopoles is formed from individual lengths of co-axial cable. In the access configuration, these lengths of co-axial cable are preferably substantially aligned with the feed cable. Preferably, the monopoles are arranged to open into a circumferential array in order to treat the oesophagus wall. In other words, the lengths of co-axial cable are at an angle to the feed cable in the treatment configuration. The monopoles may be arranged around a part or all of the circumference. Preferably the distal ends of the monopoles are rounded to prevent damage to the tissue.

Another problem addressed by the present invention is how to access the treatment area. It is desirable to avoid open surgery. It is therefore preferable for the probe of the present invention to be inserted with or as part of an endoscope. For example, the flexible patch antenna array described above may be mounted onto a flexible (or semi-flexible) co-axial feed cable for insertion into the oesophagus through an endoscopic tube (or the instrument channel). Equally the aligned position of the monopole or paddle arrangements is adapted to allow insertion through an endoscope.

Preferably, the probe has an access configuration suitable for positioning the probe in a location for treatment and an open/close mechanism for switching (e.g. causing physical transformation of) the probe between the access configuration and the treatment configuration. For example, in the access configuration, the probe may be insertable through an endoscope. The probe preferably has a diameter suitable for insertion down a throat (e.g. via the mouth or nose) into the oesophagus. Preferably the diameter is less than 5 mm; more preferably less than 3 mm. In order to fit inside an endoscopic tube, the diameter is preferably 2.8 mm or less to enable use of a standard endoscope

As mentioned above, the probe may be attached at one end of a flexible semi-rigid feed cable (preferably co-axial cable) that is arranged to provide power to the feed structure of the probe. The feed cable is preferably configured to be suitable for insertion through an endoscopic tube. Thus, its diameter is preferably less than 3 mm, more preferably in the range 2.2 to 2.8 mm in order to be small enough to fit within a conventional endoscopic tube yet large enough to carry the requisite power to the probe with a minimal amount of power loss along the cable. The feed cable is preferably 0.5 to 1.5 m in length.

The probe is preferably controllably switchable between the access configuration and the treatment configuration. The substrate is preferably radially expandable from the access configuration to the treatment configuration such that, when in use in the treatment configuration, the plurality of radiating elements are brought into close proximity or contact with the oesophageal wall. This may improve the effectiveness of the treatment because the field is generally more uniform closer to the radiating elements.

The radially expandable substrate may be achieved using the flexible tube (comprising a rolled sheet) described above. The flexible tube may be mountable on a carrier rod which is e.g. attached to the semi-rigid feed cable, the flexible tube having axial slits in its surface, which slits define substrate strips therebetween that are movable radially outward when the ends of the tube are axially moved towards each other. The probe may thus have an appearance resembling a Chinese lantern, whereby moving the axial ends of the flexible tube towards each other causes the separate strips linking them to bulge outwards. The radiating elements are preferably located at the middle of the strips so that they are at the most radially extended point.

Preferably, the open/close mechanism includes an axially movable control portion arranged to move the axial ends of the substrate tube relative.to each other. The mechanism may therefore control opening and closing of the Chinese lantern structure. The open/close mechanism may be mounted e.g. on the carrier tube or on the semi-rigid feed cable.

In an alternative arrangement, the flexible tube may be mountable on the carrier rod over one or more radially extendible members which are arranged to push the tube outwardly from a collapsed state in the access configuration to an expanded state in the treatment configuration. The flexible tube may have defined areas, e.g. lines of weakness where it is more likely to crease or fold. The lines of weakness may be arranged to provide uniform, e.g. symmetrical folded configuration for the flexible tube in its collapsed state. The flexible tube may be elastically expandable.

The radially extendible members may be springs attached to the carrier rod, and the open/close mechanism may include an axially movable control portion attached to the springs which is arranged to control the radial distance that the springs extend away from the carrier rod. The springs may therefore controllably push outward against the collapsed flexible tube to cause it to move into its expanded state. The flexible tube is preferably biased into its collapsed state so that when the springs retract, the flexible tube returns to its collapsed state. A further arrangement may comprise a plurality of sleeves (preferably two) and pull wires or chords. The pull wires are preferably arranged to move the sleeves relative to the feed cable. Preferably they are mounted co-axially on that cable. The patch antennas may be provided on radially extendable springs attached to the carrier rod and constrainable by the sleeve. Thus, the springs may be constrained by a first sleeve when the probe is in the access configuration. When in the treatment position, the first sleeve can be moved relative to the carrier rod by the pull wires to release the springs so that the radiating elements (patch antennas) move into a treatment configuration. After treatment, a second sleeve may be moved relative to the carrier rod to constrain the springs again in order to permit removal of the probe. The sleeves preferably constrain the springs to have a smaller radial spread than when they are in the treatment configuration. These sleeves are preferably made from non-metallic material. The springs on which the radiating elements are mounted are preferably made from nitinol. The pull wires may also be made from non-metallic material and can be located inside another sleeve that runs along the feed cable. This can prevent loose wires from causing problems.

The mechanism for controllably opening and closing the probe provides another independent aspect of the present invention. Accordingly, that aspect may provide a device for radially expanding a flexible tubular substrate, which substrate is attached to an axially extending rod and has an array of antenna patches mounted on its outer surface, wherein the device includes: driving means mounted on the rod; and a sleeve axially movably mounted on the rod, the sleeve being movable along the rod by the driving means to cause radial expansion of the tubular array.

Preferably, the driving means includes a coil of wire around the rod and a power supply for carrying current to the coil. Current in the coil produces a magnetic field, which is preferably utilised to cause the axial movement required to open the tubular array. For example, the coil may be mounted on a housing which is fixed to the rod in an overlapping relationship with the sleeve, whereby the sleeve is movable by a force due to the magnetic field produced when current flows through the coil. Preferably, the sleeve is located between the coil and the rod, and has a higher relative permeability

($\mu_r$) than the rod. In this case, the sleeve acts as a flux multiplier; the different effects of the magnetic field on it compared with the rod cause it to move along the rod. The sleeve can be made from any material so long as it has a higher $\mu_r$ than the rod. Steel may be used.

Alternatively, the coil may be fabricated on to a flexible printed circuit board (PCB), i.e. a circuit board having a flexible and preferably thin substrate. This may enable the thickness of the device to be less than a conventional wire coil. It may also allow the winding density to be increased. The PCB coil may be constructed by fabricating a series of parallel lines on to the substrate surface, each line being offset from its neighbour by one line width. The lines could then be electrically joined together e.g. by soldering or by conductive mechanical means to form a continuous coil or solenoidal winding.

Preferably, the device is insertable through an endoscope. As explained above, in conventional terms this means it preferably has a maximum outer diameter of 2.8 mm.

Preferably, the device includes guide means arranged to cause radial expansion of the flexible substrate, e.g. in the case where the flexible substrate is biased into a collapsed (not expanded) state. The guide means may include radially extendible elements coupled to the sleeve and arranged to extend radially outwardly when the sleeve moves in a first direction along the rod in order to cause the flexible substrate to expand. The radially extendible elements may be leaf springs attached to and extending along the rod, such that movement of the sleeve in the first direction allows the springs to extend radially away from the rod, and movement of the sleeve in a second direction opposite the first direction pulls the springs back towards the rod. An arrangement using nitinol wire to control the springs is also possible.

Alternatively, a manual open/close mechanism may be provided. For example, a second rod may be inserted into the endoscope alongside the rod carrying the radiating elements. The second rod may have a distal end attached to the radiating elements so that they can be brought into the treatment configuration when the second rod is moved relative to the carrying rod.

The whole treatment apparatus provides a further aspect of the present invention. According to this aspect, there is provided apparatus for ablating oesophageal wall tissue, the apparatus including: a controllable source of microwave radiation having a stable output frequency; a probe connected to the source of microwave radiation, the probe having an antenna arrangement (e.g. as described above) suitable for insertion into the oesophagus and arranged to emit a microwave radiation field; and a controller arranged to control the amount of energy delivered by the microwave radiation to the tissue to be treated. The apparatus is thus preferably arranged to deliver a controllable supply of microwave energy to the probe in order that a stable (uniform) radiation field is emitted to treat the oesophageal wall.

The probe is preferably a probe according to the first aspect set out above. In other words, the probe is preferably insertable into the oesophagus and is shaped to emit a microwave radiation field that penetrates the oesophagus wall to enable a region of controlled thermal damage to be produced.

Preferably, the controller includes a control unit arranged to generate a target energy dose (total amount of power delivered over time) to be delivered to the oesophageal wall. The target energy dose may be selectable to cause thermal damage to the surface layer of the oesophageal wall. Preferably, the target energy dose is selected to cause ablation or controlled thermal damage to the oesophagus wall. In other words, different types of treatment can be delivered by selecting a target energy dose. Moreover, the type of treatment can be further refined once a dose is set, e.g. by selecting a particular configuration of energy pulses to deliver the dose. For example, to perform "oesophageal reflux" treatment, a target energy dose and a pre-programmed train of pulses to deliver that dose may be selected or calculated by the control unit. Different factors e.g. diameter of oesophagus, oesophageal wall structure (which may depend on age, lifestyle, etc.) may be taken into account by the control unit when generating the target energy dose.

Preferably, the controller is arranged to ensure that the target energy dose is delivered to the oesophageal wall. Preferably, the controller includes a detector for detecting a power level of microwave radiation provided to the probe, the detected power level being used to calculate the amount of energy delivered to the oesophageal wall. The detector may include a forward directional coupler for detecting power delivered from the microwave radiation source to the probe. Furthermore, the detector may include a reverse directional coupler for detecting power reflected from the oesophageal wall back through the probe. The power delivered to the oesophageal wall may therefore correspond to the difference between the forward radiation supplied to the probe and the reflected radiation detected at the reverse directional coupler. The controller is therefore preferably arranged to calculate the power delivered to the oesophageal wall in order to ensure that the target energy dose is delivered. The difference between the forward and reflected power is typically referred to as "net" power delivered into target tissue.

The controller is also preferably arranged to ensure that the output power level follows the demanded power level to within a specified error limit to compensate for output power drift e.g. due to temperature variation. The controller may also be arranged to measure fault conditions (e.g. power cable disconnection, antenna (probe) disconnection, cable breakage, output power exceeding the demand, power supply variations or voltage rails going down); to flag up that an error has occurred; and, where necessary, take remedial action, e.g. by stopping power output. A second controller (or microprocessor) may be used to monitor said fault conditions; said second controller is often referred to as a "watchdog".

The apparatus preferably includes a power amplifier connected between the source of microwave radiation and the probe, the power amplifier being arranged to increase an output power level of the source to a power level suitable for treating the tissue. The microwave source may be any kind that delivers a stable frequency. Preferably the frequency is contained within the region of the electromagnetic spectrum known as the super high frequency (SHF) region, e.g. 14-15 GHz. The delivered frequency is stable, preferably limited to a band of a few kHz (e.g. a bandwidth of 10 kHz). The microwave source may derive its stable signal from a stable reference source, e.g. a temperature stable crystal oscillator with a fixed frequency, e.g. in the range 1-100 MHz, preferably 5-50 MHz. Preferably, the microwave source is a phase-locked dielectric resonator oscillator (DRO). Typically, the output power from such stable oscillators is not high enough to cause an effect on oesophageal wall tissue. It is therefore necessary to amplify the power between the source and the treatment probe.

In order to accurately determine the energy level delivered to the tissue to be treated, the amplification is preferably carefully controlled by the controller. Preferably, the controller includes a power level setter for controlling the output power level of the source that is provided to the input of the power amplifier. The power amplifier may have a fixed gain (e.g. 50 dB), so controlling (and preferably varying where necessary) the input power level is one way of controlling the output power level of the power amplifier. The power amplifier may have a variable gain. Alternatively or additionally, the power supply to individual transistors may be controlled in order to vary the gain, although there are limits to the range of adjustment possible with this technique.

The power level setter may be arranged to determine the output power level of the source provided to the input of the power amplifier on the basis of the detected power level of radiation provided to the probe.

Alternatively, the power level setter may be arranged to determine the output power level of the source provided to the input of the power amplifier by comparing the detected power level with a target power level derived from the target energy dose.

Preferably, the power level setter includes a signal attenuator for varying the power level input to the power amplifier. The attenuation level of the signal attenuator is preferably controllable by the controller on the basis of any difference between the target power level calculated from the target energy dose, and a detected power level of radiation being delivered to the oesophageal wall.

The apparatus may include a reflected power monitor arranged to recognise certain behaviour in a reflected signal received back from the antenna and enable action to be taken automatically in response to the recognised behaviour. The behaviour in the signal may be indicative of a condition in the tissue being treated. For example, the signal may indicate that the impedance of the tissue is not changing, which may mean that the power delivered is insufficient for effective treatment. The power level may be increased automatically or manually in response to this recognised behaviour. In another example, this arrangement can be used to reduce or prevent the phenomenon or tissue 'spitting' that can occur during treatment. Tissue 'spitting' or 'popping' is thought to be caused by pressure building up where a energy emitting surgical instrument (e.g. probe) is inserted into tissue. The combination of pressure and energy from the instrument can cause small bits of tissue to be removed from the treatment site.

The behaviour of the reflected power can indicate in advance when a spit event is about to occur. It may be possible to prevent the spit event from occurring if suitable action is taken in response to the relevant behaviour.

Thus, the reflected power monitor may be arranged to detect a signature event in the reflected signals detected by the reflected radiation detector, and a power level adjuster may be connected between the source and antenna and arranged to automatically adjust a power level of the microwave radiation signal received by the antenna if the monitor detects a signature event.

The signature event may be any detectable behaviour in the reflected signal. For example, it may be a certain rate of change of reflected power or a constant level of reflected power for a certain time slot or duration. The signature event may be derived from behaviour in the reflected power, e.g. the reflected power may be used to determine changes in the impedance of the tissue; these changes may indicate the signature event. If the arrangement detects an event indicating that the antenna is held in one place for too long (e.g. a constant voltage indicative of a well matched condition is detected) then the power can be reduced to reduce or prevent collateral damage.

The reflected power monitor may be arranged to detect a rapid voltage spike in the reflected signal. For example, the monitor may include a differentiator arranged to measure a value of dv/dt (change of voltage with time) for the reflected signals. The differentiator may be arranged to compare the measured value to a threshold value, whereby the signature event is a value of dv/dt that is higher than a threshold. This arrangement may be used to detect tissue 'spitting', which the inventors have found is preceded by a voltage spike with a sharp rise or fall. The apparatus may continuously monitor the reflected power during treatment and if the signature event (value of dv/dt above the threshold) is detected, the power level may be arranged to immediately reduce the power level from a first value to a second value. Thus, the apparatus may back off (or reduce) the power level as soon as the signature (signal) that is known to lead to a 'spit' is observed. The first value of the power level may be one or more orders of magnitude greater than the second value of the power level.

The differentiator (e.g. slope detector differentiator) may be implemented in an analogue manner, i.e. using discrete operational amplifiers, signal comparators, an arrangement of capacitors and resistors and MOSFET switches, or using digital components, e.g. a computer or a DSP unit.

The threshold may be adjustable e.g. to enable a sensitivity to tissue spitting to be selected.

The power level adjuster may be arranged to ramp the power level back to the first value in a recovery time period after the reduction in power level. In practice, the power may need to be ramped back up relatively rapidly to permit treatment to continue without substantial instrument downtime or to ensure that the overall patient treatment time is not excessive. For usage in tumour ablation, it must be ensured that critical temperatures within the tissue are reached in order to ensure that all of the cancerous tissue/cells has/have been totally destroyed. The recovery time period may therefore be 100 ms or less.

The reflected radiation detector may be selected to be sensitive to the changes in the reflected signal which represent the monitored behaviour. Thus, if a diode detector is used e.g. connected to a coupled port of a directional coupler connected between the source and the antenna, then its rise/fall time may be selected to capture the signature event. For example, the detector may be a diode detector having a rise/fall time of 1 $\mu$s or less to capture the voltage spike associated with tissue spitting event that may exhibit a rise/fall time of 10 ms. In one embodiment, a tunnel diode based detector with a very fast pulse response may be used, e.g. product number ACTP1505N from Advanced Control Systems.

The power level adjuster may comprise an impedance adjustor connected between the source and the antenna. The impedance adjustor may also be used in an impedance matching arrangement, wherein the detector may be arranged to detect the magnitude and phase of the reflected signal and the impedance adjustor may have an adjustable complex impedance that is controllable based on the detected magnitude and phase. In this arrangement the impedance adjustor may therefore be arranged to match the impedance of the apparatus to the impedance of the load (tissue) to enable efficient power transfer. The impedance matching may be dynamic, e.g. adjustment may occur automatically in real time. When a signature event is detected by the monitor, the impedance matching may be overridden by the response to that signature event.

The reflected power monitor may also be arranged to provide user information e.g. to guide the surgeon during treatment. For example, the monitor may be arranged to emit an audible or visual signal when a signature event is detected. The audible or visual signal may be representative of the detected event. The audible signal may be any of a range of sounds or a digitally synthesised voice.

Preferably, a power isolation device is connected between the probe and the power amplifier to protect the amplifier from high levels of radiation reflected back through the probe from an impedance mismatch at the tissue. Preferably, the power isolation device has an isolation value selected to protect the amplifier from reflected radiation occurring due to a predetermined range of probe/tissue mismatch conditions and the maximum output power available from the amplifier.

Preferably, the controller includes a modulator for adapting the microwave radiation signal before it is supplied to the tissue to be treated. Preferably, the modulator is arranged to adapt an output signal of the source of microwave radiation. More preferably, modulation occurs before amplification.

The signal is preferably modulated to improve the transfer of energy to the tissue to be treated. Preferably, the modulator is arranged to produce a pulsed signal to be provided to the probe. A pulsed signal may permit a higher power level to be applied over a shorter period of time to achieve a predetermined energy dose. Pulsing in this way is effective where the desired form of energy transport from the probe to the tissue to be treated is radiation. Disadvantageous effects associated with conduction, such as conductive heating of the probe and its feed cable, can therefore be mitigated.

The modulator may be a pin diode switch. Thus, the apparatus may be arranged to deliver controlled programmable doses of energy using a stable source oscillator (e.g. having a frequency stability of less that 10 kHz at 14.5GHz), a PIN diode absorptive switch modulator with breakthrough protection (to prevent switch frequency components from escaping to the amplifier or microwave source), a PIN diode absorptive attenuator (power level setter), a solid state amplifier line-up (e.g. pre-amplifier and main power amplifier) and directional couplers to measure forward and reflected power levels. The controller may include a first microprocessor for operating the above microwave components, and (optionally) a second microprocessor to monitor fault conditions, i.e. malfunction of first microprocessor or power supply line faults. Energy flow through the system into the patient may be controllable via a footswitch. The footswitch may be of the simple on/off (i.e. normally open (NO) or normally closed (NC)) type. Preferably, a DC isolation path circuit is included to ensure that no DC path exists between the generator and the user/operator.

The above apparatus may enable fine precision control of the delivered power level in either a pulsed or a continuous mode of operation. In pulse mode operation, duty cycles are selected to provide optimal tissue effect. Pulse mode enables high pulse power levels to be maintained for short periods of time, for example, a treatment structure that may be operated at 50 W in continuous mode operation, may be operated at 200 W for short durations of time, for example 50 ms with a duty cycle of 1:10. This operation offers the significant advantages where the energy transport mechanism is radiation. This form of operation also offers the advantage of extremely fast treatment time and eliminates problems associated with conductive heating of treatment antenna probes, flexible semi-rigid cable feed assemblies and the flexible co-axial cable assembly. It is also possible for the controller to send bursts of pulses with pre-selected pulse amplitudes and widths (e.g. 10 pulses of width 10 ms, 50% duty cycle and amplitude 10 W followed by 20 pulses of width 2 ms, 20% duty cycle and amplitude 40 W). Such pulse sequences may be selected to treat and are particularly suitable for treating a certain condition. It is also possible to use the microprocessor to generate a variety of pulse shapes, e.g. square, ramp, triangular, sine, etc.

Preferably, the controller is arranged to monitor the source of microwave radiation to prevent erroneous signals from reaching the probe. For example, the controller may check that the frequency of delivered radiation does not deviate outside a predetermined bandwidth associated with the fixed frequency. It may be preferable to use a second microprocessor to monitor this function.

Preferably, there is an isolation barrier between the probe and the rest of the apparatus, the isolation barrier being arranged to prevent direct current from flowing through the probe and into the patient.

In a preferred embodiment, the apparatus preferably comprises a stable frequency source, a pulse modulator with DC and low frequency breakthrough suppression, a wide range digitally controlled attenuator, a pre-amplifier and power amplifier with reflected power level protection and a DC system to user isolation barrier, forward and reflected power monitors and detectors, a flexible co-axial cable assembly, a flexible semi-rigid cable assembly (suitable for insertion into the oesophagus) and a probe comprising: a patch antenna array fabricated onto flexible substrate material, a means of opening and closing said patch antenna array within the oesophagus, a microprocessor for controlling the microwave radiation producing components, and an on/off footswitch circuit for operating the apparatus with a DC isolation barrier between the system and the external footswitch cable.

Thus, whilst the discussions above concern the use of the present invention which is not part of the invention in treating the oesophagus, the present invention may additionally relate to a treatment system using one or more radiating antenna elements on a probe to produce controlled microwave radiation at frequencies that lie within the super high frequency (SHF) region of the electromagnetic spectrum to enable controlled ablation, or controlled tissue destruction, or controlled or thermal damage, of various other tissue structures forming hollow tube structures within the human or animal body. However, this invention is particularly relevant to a treatment system with multiple patch antenna probe structures fed from a flexible semi-rigid co-axial feed and interstitially inserted into the oesophagus e.g. to treat stomach condition.

Thus, in another aspect, there is disclosed a method, which is not part of the invention, of treating oesophageal wall tissue with microwave radiation, the method including: inserting a probe in an oesophagus, the probe having a tubular antenna arrangement; connecting a source of microwave radiation having a stable output frequency to the probe, whereby the tubular antenna arrangement emits a circumferential microwave radiation field; controlling the amount of energy delivered by the microwave radiation to the tissue to be treated. Preferably, the emitted radiation field has a substantially uniform energy density where it penetrates the oesophagus wall. Such an arrangement desirably causes a substantially uniform lesion along the oesophageal wall, which is beneficial for treatment. As discussed above, a target energy dose is preferably delivered to the oesophageal wall. The target energy dose is preferably selected to cause ablation of the oesophageal wall. Such a method may be generally applied in the field of hollow tube pathology.

## BRIEF DESCRIPTION OF THE DRAWINGS

Examples of the present invention are now described with reference to the accompanying drawings, in which:

Fig. 1 is a schematic block diagram of apparatus for ablating oesophageal wall tissue which is an embodiment of the invention;
Fig. 2 depicts the location of the oesophagus in the human body;
Fig. 3 is a more detailed block diagram of the apparatus shown in Fig. 1;
Fig. 3A illustrates schematically the component line-up which delivers power to the tissue;
Fig. 3B is an exaggerated illustration of a probe configuration;
Fig. 4 illustrates a flat patch antenna array for a probe which is an embodiment of the invention;
Fig. 5 shows the patch antenna array of Fig. 4 in a rolled configuration;
Fig. 6 illustrates a parallel feed structure for delivering power to a 1-D patch antenna array in a probe which is an embodiment of the invention;
Fig. 7 illustrates a parallel feed structure for delivering power to a 2-D patch antenna array in a probe which is an embodiment of the invention;
Fig. 8 illustrates a series feed structure for delivering power to a 2-D patch antenna array in a probe which is an embodiment of the invention;
Fig. 9 shows the patch antenna array of Fig. 8 in a rolled configuration;
Fig. 10 shows a cross-section of a radiating patch with the feed line electromagnetically coupled to the radiating patch;
Figs. 11A and 11B show a slotted line antenna (another 1-D patch antenna array) for use in a probe which is an embodiment of the present invention;

Fig. 12 shows the results of a computer simulation of the radiation field strength produced by the antenna array shown in Figs. 11A and 11B;

Fig. 13 shows a schematic view of a probe in an access configuration being inserted into an oesophagus;

Fig. 14 shows a schematic view of a probe in a treatment configuration inside an oesophagus;

Fig. 15 shows a side view of a open/close mechanism for the probe illustrated in Figs. 13 and 14;

Fig. 16 shows a schematic view of a device for opening the probe shown in Figs. 13 and 14 which is an embodiment of the present invention;

Figs. 17A and 17B show respectively a cross-section view of a collapsed and an expanded probe which is an embodiment of the present invention;

Fig. 18 shows a flat antenna array for a probe with slits similar to a Chinese lantern which is another embodiment of the present invention;

Fig. 19 shows the antenna array of Fig. 18 in a rolled access configuration;

Fig. 20 shows the antenna array of Fig. 18 in a rolled treatment configuration similar to a Chinese lantern;

Fig. 21 shows a schematic view of a probe having the antenna array of Fig. 18 opened up in the form of a Chinese lantern inside an oesophagus;

Fig. 22 shows a microstrip probe configuration that is another embodiment of the invention;

Fig. 23 shows detailed dimensions of another microstrip probe according to the invention;

Fig. 24 shows a probe formed on a surgical balloon, which is yet another embodiment of the invention;

Fig. 25 schematically illustrates the impedance matching condition of the probe of Fig. 24;

Fig. 26 is a cross section of the co-axial feed cable shown in Fig. 24;

Fig. 27 is a close up view of the quarter wave transformer shown in Fig. 24;

Figs. 28A and 28B illustrate another probe formed on a surgical balloon, which is yet another embodiment of the invention;

Fig. 29 illustrates an alternative position for an impedance matching transformer in the feed structure; and

Fig. 30 shows an embodiment of a split feed structure suitable for providing power to a plurality of slot antennas on a surgical balloon.


## DETAILED DESCRIPTION; FURTHER OPTIONS AND PREFERENCES

### Treatment Apparatus

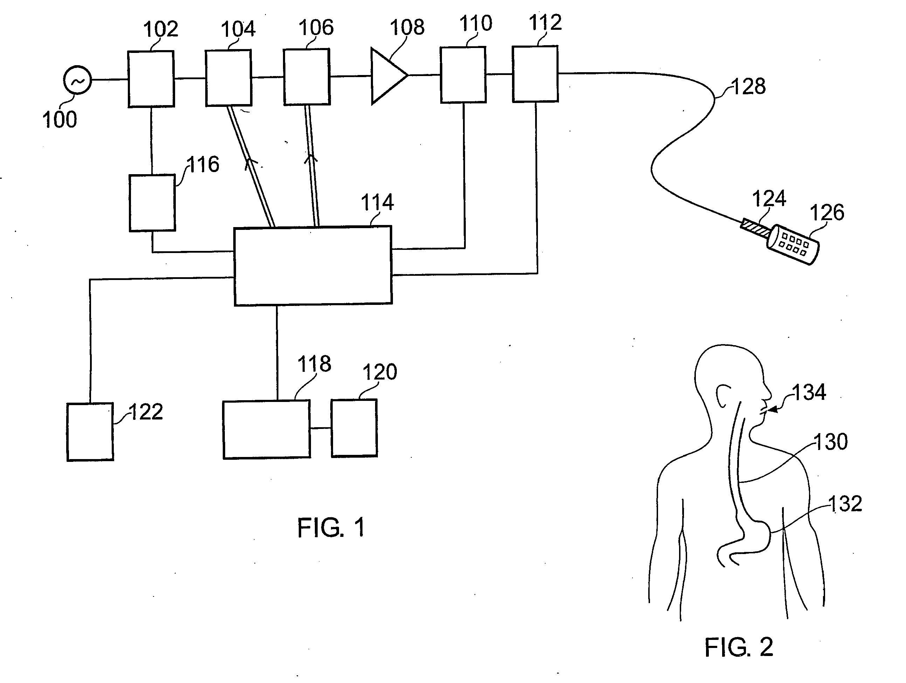

Firstly, an overview of an embodiment of the whole treatment system will be given, with reference to Figs. 1 to 3.

Fig. 1 illustrates the basic structure of the apparatus for treating the oesophageal wall. A stable microwave signal is produced by a microwave source 100. This signal is split into two by splitter 102.

One part of the split signal is sent to a monitor 116 which is configured to check for low level power coming out of the stable source 100 i.e. if the power level is below a threshold value, set for valid instrument operation, then an error condition will be flagged up. The results of the check by monitor 116 are provided to microprocessor 114, which is arranged to control the operation of the apparatus, and can stop treatment if the monitor 116 detects something wrong with the signal provided to it.

The other part of the split signal is used for treatment. It is firstly sent to a signal modulator 104, which adapts the signal into a shape suitable for treatment. This may be a sequence of rectangular pulses or continuous wave energy e.g. controlled by a foot switch. The signal then passes through an attenuator 106, which is arranged to adjust its power level (amplitude) to a level suitable for amplification. The signal modulator 104 and attenuator 106 are controlled by the microprocessor 114 on the basis of measurements taken after signal amplification. The microprocessor 114 is arranged to adjust the signal modulation at modulator 104 and signal attenuation at attenuator 106 to ensure that a predetermined amount of power is delivered to the oesophageal wall. The position of modulator 104 and attenuator 106 is interchangeable.

After the attenuator 106, the signal is amplified by amplifier 108 to a level that is suitable for treatment e.g. to effect controlled ablation of the oesophageal wall. After the amplifier 108, the signal is provided to a treatment probe 126 via a co-axial feed cable 128 and feed structure 124. The probe radiates a microwave field which penetrates and controllably ablates the oesophageal wall.

A forward power coupler 110 and a reverse power coupler 112 are located between the amplifier 108 and probe 126 to detect a portion of the power delivered to the probe and the power reflected from the tissue back through the probe respectively. The detected signals are provided to the microprocessor 114, which uses them to calculate the amount of power actually delivered to the oesophageal wall.

A user control interface 118 and display 120, allow a user to program the apparatus, e.g. by specifying a target energy dose to be delivered to the oesophageal wall. The microprocessor 114 can adjust the signal delivered to the probe 126 to comply with the specified target energy dose on the basis of the inputs from the forward and reverse power couplers 110,112 and the user control interface 118. Of course, the apparatus may also permit manual application of power in addition to this automated mode. For example, the user/operator (e.g. surgeon) may set a desired power and apply power until the desired thermal damage has been achieved.

Referring to Fig. 2, the probe 126, feed structure 124 and feed cable 128 can be inserted via an endoscope through the nose or mouth 134 into the oesophagus 130, to any location above the stomach 132. Once in position, the apparatus can be switched on and off by the user using footswitch 122, which is connected to the microprocessor 114. The operation of the unit could be said to be under diathermic control.

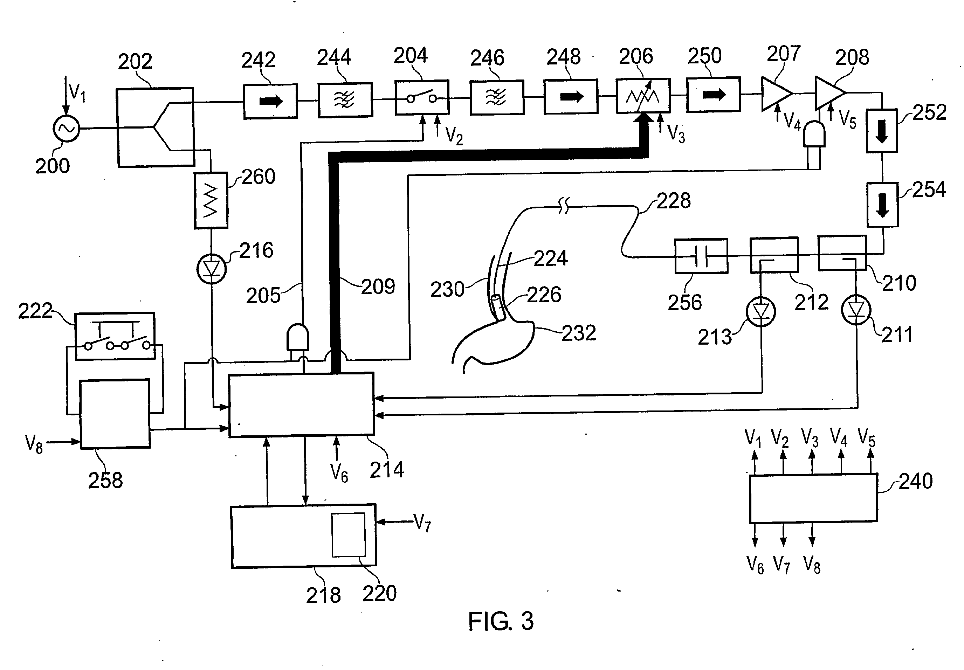

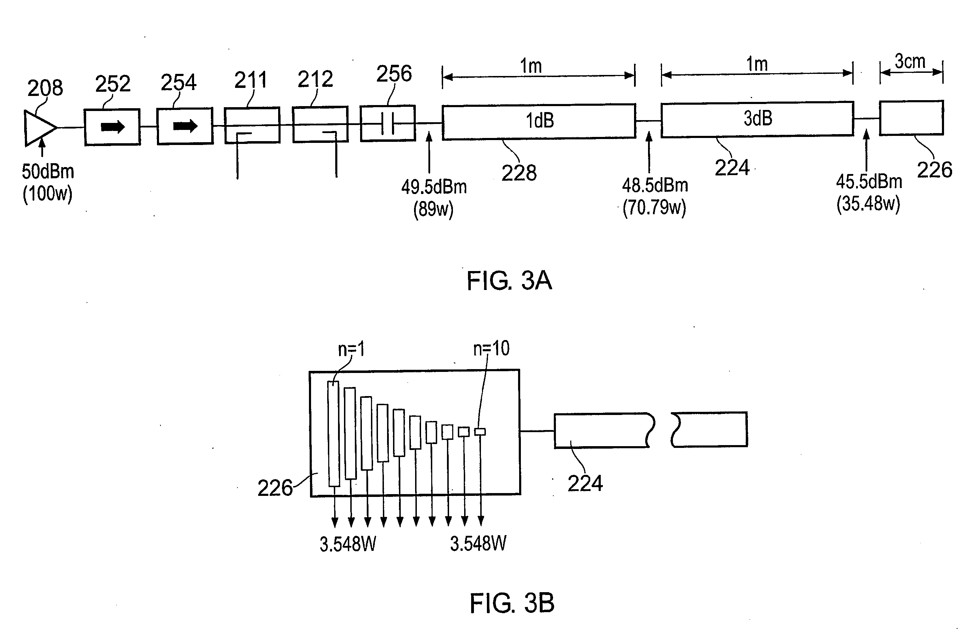

Fig. 3 shows the apparatus of Fig. 1 in more detail. Here, the microwave signal is provided by a stable frequency source 200, which provides a signal at a single frequency contained within the super high frequency (SHF) region of the electromagnetic spectrum and, more specifically, at 14.5GHz (with the bandwidth limited to a few kHz). The stable frequency source 200 shown here takes the form of a phase locked dielectric resonator oscillator (DRO), which contains a reference signal to which the frequency stability of microwave source 200 is derived; the source of said reference signal (not shown) may comprise a temperature stable crystal oscillator, operating at a frequency in the range of between 1MHz and 100MHz, but more preferably between 10MHz and 50MHz. Said stable crystal oscillator may be used for system timing functions (so could be connected to microprocessor 214), and also as the oscillator signal used to excite a pulsed transformer that forms a part of a specific embodiment, used in the current invention, for the footswitch DC isolation barrier circuit 258. Other frequency sources, such as voltage controlled oscillators (VCOs) or Gunn diode oscillators may also be used, but it is preferable to use a DRO in the present invention due to the preferred frequency of operation.

The stable frequency source 200 is connected to the input port of a 3dB, 0° power splitter 202. The purpose of splitter 202 is to divide the power produced by source 200 into two equal ratios without introducing a phase change.

The first output from splitter 202 is connected to the input of a first signal isolator 242, and the second output from splitter 202 is connected to the input of attenuation pad 260. The purpose of attenuator pad 260 is to limit the signal level incident at the input to a detector diode 216 to prevent damage caused by too high a signal level. In practice, attenuation pad 260 may not be required (i.e. it is not essential to the functioning of the invention); it is included in the illustrated embodiment as a precautionary measure. The output of detector diode 216 is input into microprocessor 214 where the signal is used to monitor the status of the frequency source 200. Should the signal indicate that signal source 200 is functioning improperly, then microprocessor 214 will flag up that an error has occurred and the system will take appropriate action, i.e. an error message will be generated, and/or the system will shut down. It may be appropriate for the system to display a message indicating that a service engineer, or technician should be consulted.

The purpose of first signal isolator 242 is to prevent any mismatched signal present at the input of first modulation breakthrough blocking filter 244 causing frequency changes at source 200, due to, for example, load pulling, or another condition that may affect the signal generated by signal source 200. In practice, isolator 242 may not be required if the input port of filter 244 is well matched, but isolator 242 is included as a precautionary measure. The output from first signal isolator 242 is connected to the input of first modulation breakthrough filter 244, whose function is to prevent frequency components contained within any fast switching signals produced by PIN diode modulation switch 204 from getting back to the stable frequency source 200 and affecting its operation.

The output of first modulation breakthrough filter 244 is connected to the input of said PIN diode modulation switch 204, whose function is to modulate the signal produced by stable frequency source 200 to enable the system to operate in pulsed mode, whereby the duty cycle, pulse width, and (if wanted) pulse shape can be modified using the user control and display unit 218,220 and the microprocessor unit and signal conditioning circuits 214. The input control signal 205 to the modulation switch 204 comes from the microprocessor unit and signal conditioning circuits 214. This control signal 205 may be a transistor-transistor logic (TTL) level signal; other signal formats (e.g. emitter coupled logic (ECL)) are possible.

The output from the modulation switch 204 is connected to the input of a second modulation breakthrough blocking filter 246, whose function is to prevent frequency components contained within any fast switching signals produced by PIN diode modulation switch 204 from getting into the pre-amplifier 207 and power amplifier 208 stages and causing, for example, signal distortion, erroneous output power levels, or damage to these units through, for example, the manifestation of output power stage oscillation, or signal overdrive caused by one of the harmonics contained within the switching signal occurring at the same frequency as that of the signal generated by the frequency source 200 or a signal that is within the bandwidth of the amplifiers 207,208.

A practical implementation of the breakthrough blocking filter may simply be two co-axial to waveguide adaptors using rectangular waveguide sections, where frequencies lower than the cut-off frequency of the waveguide section will be blocked, hence the waveguide section acts as a high pass filter. In this arrangement, the two co-axial to waveguide adaptors will be connected back to back using, for example, four screws going through waveguides flanges.

The output from second modulation breakthrough blocking filter 246 is connected to the input of a second signal isolator 248, whose function is to provide isolation between the PIN diode attenuator 206 and the second modulation breakthrough blocking filter 246. The second signal isolator 248 is inserted for good design practice and could in fact be omitted from the apparatus without causing degradation or damage to the microwave sub-assembly.

The output from said second isolator 248 is connected to said PIN diode attenuator 206, whose function is to enable the system power level to be controlled by changing the level of signal attenuation using input control signals 209 produced by microprocessor unit and signal conditioning circuits 214. It is preferable for the variable attenuator control signal 209 to be a digital signal consisting of a plurality of control lines that are switched between the logic states of 0 and 1. Said control signals may be TTL level signals. It is preferable for PIN diode attenuator 206 to be an absorptive type attenuator rather than a reflective type. As an example, a possible candidate for attenuator 206 is an 8-bit, 64dB variable digital attenuator that can operate between the frequency range of 8 GHz and 18GHz, available from Advanced Control Components Inc. (part number ACAT-B181). Typical circuit topologies incorporate PIN diode switched-bit attenuator sections or analogue attenuators driven by analogue-to-digital (A-to-D) converters.

The output from PIN diode attenuator 206 is fed into the input of third low power signal isolator 250, whose function is to isolate (or attenuate) any reflected signal present at the input of pre-amplifier 207 from getting through to the output of PIN diode attenuator 206. Again, this unit is included for good measure; it can be omitted if the match between the PIN attenuator 206 and the input to the pre-amplifier 207 is adequate.

The output from the third signal isolator 250 is connected to the input of signal pre-amplifier 207, whose function is to amplify the signal to a level that is acceptable for driving the input to the subsequent power amplifier stage 208. The preamplifier 207 may provide a gain of between 10dB and 40dB necessary to drive the power amplifier 208. The preamplifier 207 may come in the form of a single miniature microwave integrated circuit (MMIC), a plurality of MMICs, a combination of MMIC(s) and discrete parts, or a plurality of discrete parts. The preferred device technology for use in the pre-amplifier is gallium- arsenide (GaAs) technology, although there are other emerging technologies that may provide viable alternatives, for example, gallium nitride (GaNi) or high electron mobility transistors (HEMTs).

The output from pre-amplifier 207 feeds the input to power amplifier 208, whose function is to boost the signal to a level that is useful for causing tissue ablation, or desiccation, or for producing controlled thermal damage to the oesophagus (or other biological tissue that is of interest). The preferred architecture for power amplifier 208 uses a plurality of GaAs power devices combined using microstrip or waveguide power combiners. As an example of the design of a possible power amplifier stage 208, four 12 W internally matched high power GaAs parts from Eudyna (part number FLM1314-12F) may be combined using three microstrip power combiners; assuming that the loss through said combiners is limited to 8 W, it would be possible to obtain 40 W of microwave power at the output of the power amplifier stage 208. As an example of a power budget for the pre-amplifier 207 and power amplifier 208 combination, if one assumes that the input power to preamplifier 207 is 0 dBm (1 mW), and two MMICs are used for that preamplifier, that produce a total gain of 40 dB, then the power amplifier 208 will need to provide a gain of 6.81 dB to compensate for the insertion loss of the power combiner network.

The output from power amplifier 208 feeds the input to fourth signal isolator 252, whose output is connected in series with a fifth signal isolator 254. Said fourth and fifth isolators 252,254 are power isolators and are used to protect the output stage of power amplifier 208 from damage caused by high levels of reflected (or reverse) power reflected back from the treatment antenna probe 226 during a mismatched condition between said antenna probe 226 and biological tissue 230. The reflected impedance may also change the matching network or the output of the power devices used in the output stage and this may cause the transistor to behave like an oscillator, thus damage may occur. The isolators help ensure that the output of the transistor "sees" 50Ω even when non-50Ω reflective impedances occur. It may be possible to omit one of the isolators 252,254 and maintain enough protection for amplifier 208, especially if the isolator exhibits high isolation. Power isolators 252 and 254 provide the combined level of isolation to ensure that the transistors used in the output stage of the power amplifier 208 cannot be damaged under any mismatch condition that may occur. Signal isolators 252,254 also prevent the amplifier from going unstable due to undesirable reflected impedances causing output mismatch.

A typical value of isolation for signal isolators 242,248,250,252,254 is 23 dB; this implies that any signal that tries to flow in the direction opposite to that of the desired direction (i.e. against the direction of the arrow) will be attenuated by 23 dB. In the instance where the two output power isolators 252,254 are placed in series, and the isolation for each isolator is 23 dB, the total isolation is 46 dB. Thus, for a signal of amplitude 46 dBm (40 W) travelling in the direction opposite to that of the arrows, the reflected signal component at the input to attenuator 140 will be reduced to 0 dBm (1 mW).

The isolators 242,248,250,252,254 can also be described as microwave circulators with a 50 Ω dump load connected to the third port of the device (this assumes that the first port is the input port and the second port is the output port. Using this view, the dump load is preferably specified appropriately to handle the levels of reflected power that may occur at that particular location in the microwave line-up.

The output from fifth signal isolator 254 is fed into the input of a first directional coupler 210, whose function is to measure a portion of the forward power generated by the microwave radiation generating line-up, which may comprise source 200, splitter 202, first isolator 242, first breakthrough filter 244, PIN diode switch 204, second breakthrough filter 246, second isolator 248, PIN attenuator 206, third isolator 250, preamplifier 207 and power amplifier 208 inclusive (less the insertion loss of series connected fourth and fifth isolators 252,254).

The output coupled port of forward coupler 210 is fed into forward power detector 211, which converts the detected microwave signal into a low frequency, or DC, signal level that is representative of the power produced by the microwave generator line-up. The signal produced at the output of detector 211 may be conditioned (filtered, amplified and DC offset) before being processed. Said signal conditioning and processing is performed using microprocessor and signal conditioning circuits 214 (it should be noted that said microprocessor and signal conditioning circuits 214 may be split up into two units, namely a digital microprocessor unit and analogue signal conditioning circuits - for convenience the two elements are shown together in Fig. 3).

The output from first directional coupler 210 is fed into the input of second directional coupler 212, whose function is to measure a portion of the power being reflected from treatment antenna probe 226. The output coupled port of 212 is fed into reflected power detector 213, which converts the microwave signal seen at its input into a low frequency, or DC, signal level representative of the power level being reflected due to an impedance mismatch between treatment antenna probe 226 and biological tissue 230. The signal produced at the output of detector 213 may be conditioned (filtered, amplified and DC offset) before being processed. Said signal conditioning and processing is performed using microprocessor unit and signal conditioning circuits 214.

The directional couplers 210,212 may come in the form of waveguide couplers, co-axial couplers, or stripline (microstrip) couplers. Waveguide couplers are often preferable due to the fact that they tend to offer the highest value of directivity (ability to differentiate between the coupled power and the power flowing in the opposite direction to the coupled power [uncoupled power]). It may be preferable to use waveguide isolators and waveguide couplers instead of co-axial isolators and co-axial couplers for the fourth and fifth isolators 252,254 and directional couplers 210,212 respectively. In one specific embodiment of the system, directional couplers 210,212 are two loop couplers contained within a single section of WR75 rectangular waveguide. Loop couplers are suitable for applications where a single frequency or narrow bandwidth is required.

The output coupled ports of directional couplers 210,212 may be designed to have coupling factors of between 10 dB and 50 dB; factors that govern the choice of coupling factor include: the ability to discern the measurement signal from the noise signal, the prevention of saturation (and damage) of forward and reflected power detectors 211,213, the ability not to significantly affect the through power of the system, and having a high enough directivity to discern the coupled signal level from the uncoupled signal level. A nominal value of coupling factor is 20 dB, which means that the main-line signal is attenuated by 20 dB, i.e. if the main-line power is 40 dBm (10 W) then the coupled power is 20 dBm (100 mW), hence through power is reduced to 9.9 W.

The forward and reflected power detectors 211,213 may take a number of forms; for example, zero bias Schottky diodes, channel Schottky diodes, biased co-axial Schottky diodes, and tunnel diodes. Tunnel diodes are particularly useful in this system due to their output level stability performance over a large range of temperatures and their fast pulse response time. For example, the ACTP1505N device from Advanced Control Components has an output capacitance of 9 pF (the low value of capacitance enables fast response times to be achieved), operation up to 18 GHz, and a sensitivity of 700 mV/mW.

The mathematical subtraction of the reflected power from the forward power can be used to determine the net power level being delivered into the biological tissue, and the energy delivered can be determined from product of said net power level and the duration, or ON time (pulsed or continuous wave), i.e. energy (in Joules) = power (in Watts) x time (in seconds). The microprocessor 214 is used to perform these calculations.

A DC isolation path between the output of the generator and the user circuit (probe 226) is created between the output of second directional coupler 212 and the proximal end of cable assembly 228 using DC isolation unit 256, which comprises a low loss dielectric material to isolate the DC path whilst allowing AC (SHF) signals to pass through. In order to prevent RF leakage caused by the thickness of the dielectric isolator 256 it may be preferable to use a quarter wave choke flange arrangement. For example, a N-male to WR75 waveguide adaptor can be used whereby a choke flange is constructed inside the face of the waveguide flange that forms a part of the waveguide to co-axial transition, or adaptor. The output from directional coupler 212 may comprise a plain flange and said waveguide to co-axial N adaptor may contain said choke. For a basic construction, a circular choke ring of 'L' cross section is used in the choke flange in order to reflect a short circuit at the junction of the two waveguide sections. This is possible due to the fact that the total length of the ring cross section is half a wavelength at the frequency of operation and since the far end is short circuited, an electrical short circuit is placed at the surface between directional coupler 212 and DC isolator 256 where a mechanical short circuit would be difficult to achieve.

The output from DC isolator 256 is an N-type co-axial connector, and is preferably the female-type. This connects to a first cable assembly 228, which is used to make the electrical connection between the output of the microwave radiation generating line up and the feed line 224 that is inserted into the oesophagus 230. The cable assembly 228 may be a co-axial cable or a section of rectangular, or circular, waveguide. When a waveguide assembly is used, it is preferable to use a flexible, or a flexible and twistable construction. The advantage of using such an assembly over a conventional co-axial assembly is that waveguide assemblies generally exhibit a lower insertion loss than the co-axial assemblies, thus generator power loss, and cable heating effects, are reduced. Low loss co-axial assemblies may still be used however. The length of the cable assembly is typically 0.5 to 1.0 m, and will have an electrically insulated jacket surrounding it. Preferably the jacket is made from a biocompatible material. The outside diameter of the first cable assembly 228 is typically between 4 and 12 mm. It is preferable for the power loss (or insertion loss) of first cable assembly 228 to be less than 1dB at 14.5GHz.

The distal end of cable assembly 228 is joined (e.g. via an SMA type connector) to a feed line 224 which is a co-axial cable assembly suitable for insertion inside the oesophagus 230 (or other suitable region of the biological system). For example, the.feed line 224 may be insertable through the instrument channel of an endoscope. The feed line 224 includes a semi-flexible semi-rigid cable, or a similar construction to facilitate user control during insertion. The feed line 224 does not have an insulating jacket. This allows an increased diameter (within the limitations presented by access requirements) which helps to reduce insertion loss through the feed structures. It may, however, be desirable for the outer conductor of 224 to be covered by a thin layer of biocompatible insulating material in order to prevent fluid from getting through the outer conductor into the dielectric layer. This may be particularly important where the outer conductor of 224 is non-continuous, which may be the case for a semi-rigid construction. The outer conductor may be coated with a thin layer of Parylene C. For example, a conformal coating of $10\mu$m in thickness may be applied. The complete assembly comprising 224 (second cable assembly) and antenna probe 226 can be coated with a conformal coating of Parylene C in order to make all parts that may be contact with a patient's tissue biocompatible and e.g. compliant with ISO 10993 (biological evaluation of medical devices). Since insertion loss is inversely proportional to the diameter of the dielectric material and the centre conductor within the co-axial cable, it is desirable to maximise the diameter of these components and, in particular, the diameter of the inner conductor. The feed line 224 preferably has a diameter of less than 5 mm. When the feed line 224 is inserted by itself, it may be covered with a biocompatible material, e.g. Parylene C. Otherwise, the feed line 224 (and attached probe 226) may be inserted through an endoscopic tube.

Impedance matching can be implemented at any or all of the following locations:

    1) The proximal end of first assembly 228
    2) The distal end of first assembly 228
    3) The proximal end of second assembly 224
    4) The distal end of second assembly 224.

Impedance matching is typically performed using co-axial quarter wave (or odd multiples thereof) transformers. By performing impedance matching at a location other than at the interface between the distal end of the cable assembly 225 and antenna 226, the ease of manufacture of the probe is increased because the locations mentioned above are more accessible than the distal end of the structure. Furthermore, the cable assemblies 224, 228 need not be 50$\Omega$ if transformers are used, i.e. lower impedance assemblies facilitate higher current densities and higher impedance assemblies facilitate higher voltages. Non 50$\Omega$ assemblies may exhibit lower insertion loss and given more flexibility to the physical geometry of the assembly.

Where large transformation is required, more than one transformer can be used at each matching location. For example, it is not usually practical to transform from 1$\Omega$ to 50$\Omega$ in a single step. However, this can be more effectively achieved using e.g. a pair of transformers which perform the transforming in steps.

Preferably, performing impedance matching at the alternative locations mentioned above means that it is not necessary to perform matching at the distal end of the second cable assembly. For example, if the characteristic impedance of the antenna structure 226 is e.g. 12.5$\Omega$, the second cable assembly 224 could be given a characteristic impedance of 12.5 $\Omega$ to match it and the second cable could be impedance matched to the distal end of feed cable 224 (whose characteristic impedance is 50 $\Omega$) using a quarter-wave transformer with a characteristic impedance of

$$\sqrt{12.5 \times 50} = 25\,\Omega.$$

The characteristic impedance of the "wave transformer" may be achieved by altering the diameter of the dielectric material in the cable assembly 224 for a distance equal to an odd multiple of quarter wavelengths.

An alternative means of replacing the impedance matching transformer between the antenna structure and the distal end of feed cable with a transformer at a location that is more accessible, e.g. at the proximal end of the feed cable, is to make the length of the transmission line between the transformer (located at the proximal end of the feed cable) and the distal end antenna an exact multiple of half the wavelength at the frequency of operation, i.e. $\dfrac{2n-1}{2}\lambda$.

Transmission line theory states that if the distance of the transmission line is an exact multiple of $\lambda/2$, then the impedance at the source end is the same as that at the load end.

In the present invention, the impedance of the load (in this case, the impedance presented by the antenna structure to the cable) is typically fixed, but is much lower than the characteristic impedance of the filled line. Thus, the length of the feed line may be made long and a transformer may be inserted at the proximal end to transform the lowest impedance to the impedance of the first cable, which is preferably 50Ω.

Due to the large phase variations at high microwave frequencies, it may be desirable to include a means to phase adjustment within the second cable assembly.

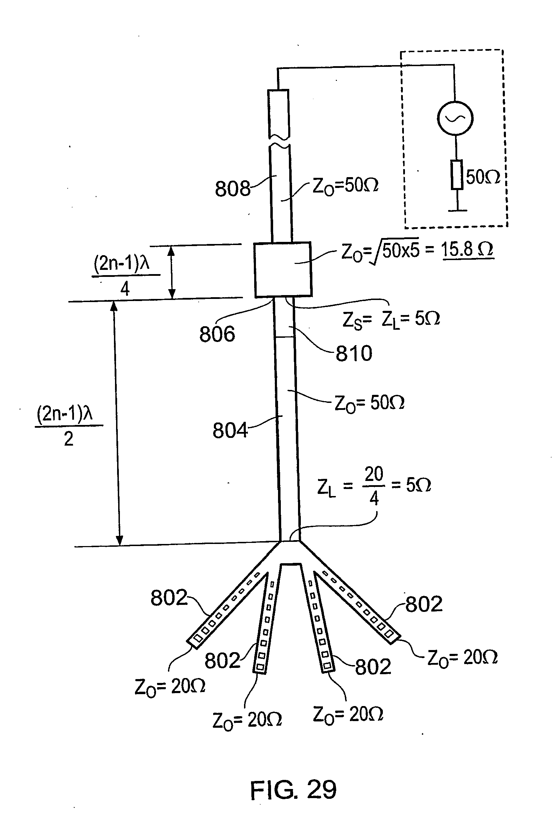

Fig. 29 shows an example of a typical load presented to the distal end of the feed cable 804. In this instance there are four paddle structures 802 connected in parallel, each with a characteristic impedance of 20Ω, giving a combined impedance ($Z_L$) of 5Ω. If the length of the feed cable 804 is adjusted to be an exact multiple of λ/2 at 14.5GHZ, then the impedance of the feed cable is theoretically transparent, hence it can be any value (although typically 50Ω cable is used for convenience). Given the above conditions, the impedance ($Z_s$) "seen" at the proximal end 806 of the freed cable is the same of that seen at the distal end, i.e. 5Ω. An impedance transformation is then performed to match the 5Ω to the 50Ω impedance of the first cable 808, i.e. a $\sqrt{5 \times 50} = 15.8\,\Omega$ transformer is required. The length of the transformer must be an odd multiple of quarter wavelength (λ/4) at 14.5 GHZ to enable the section of transmission line to act as a transformer. A phase adjuster 810 is attached at the proximal end of the feed cable 804.

Alternatively, the cable assemblies 228, 224 may be spliced together to form one single assembly.

The distal end of feed cable 224 terminates at the input of treatment antenna probe 226. The treatment antenna 226 may be constructed using a flexible low loss dielectric material with an array of patch antennas fabricated onto one side, in a manner such as to enable the radiation pattern produced to cause uniform thermal damage of oesophageal tract 230, as explained below. The patch antennas 226 are rolled up before insertion into the oesophagus 230 and opened up when inside. The radiating patches or other appropriate antenna structures may come into contact with the wall of the oesophagus 230, hence it is desirable either for those radiating patches to be fabricated using a biocompatible material or their surfaces must be coated with a biocompatible material.

The cable assemblies 224,228 may be connected to the antenna probe 226 is such a manner that the three elements together form a single use disposable item, i.e. none of these three components can be disassembled for re-use.

The length of the antenna probe may be between 1 cm and 5 cm, the upper end of the range being limited by the level of bending required to enable the probe 226 to be inserted into the treatment zone 230. The probe 226 is closed before removal. The configuration of the probe is discussed in more detail below.

The delivery of power into tissue through the apparatus described above is now explained with reference to Figs. 3A and 3B. If feed cable 224 is 1 metre in length and exhibits a loss of 3 dB, then it is conceivable that the power at the input to the feed cable 224 would be around 48.5 dBm if we assume that the maximum power from the power amplifier 208 is 50 dBm (100 W) and the total insertion loss of microwave components 252,254,211,212,256 and first cable assembly 228 is 1.5 dB. The feed cable 224 may have a characteristic impedance different from the standard 50Ω value, and use an impedance transformer at the proximal end of 224 to impedance match 224 to the first cable 228, which is typically 50Ω. The advantage of making feed cable 224 non-50Ω may be that the insertion loss can be reduced.

Thus, 48.5 dBm (= 70.79 W) may reduce to 45.5 dBm (35.48 W) at the input to antenna 226. This implies that if feed cable 224 is 1 metre in length then the loss per centimetre is 0.355 W, and if the length of antenna 226 is 3 cm, then the power per centimetre that can be launched into the tissue is 11.83 W, thus significantly greater than that lost through the feed cable.

The microprocessor unit and signal conditioning circuits 214 are used to control the operation of the apparatus. Signals from detectors 211,213,216 are filtered, amplified and have their DC levels adjusted before entering the microprocessor 214 for signal manipulation and processing. The microprocessor unit and signal conditioning circuits 214 receive input signals from the detectors 211,213,216, the user control and display unit 218,220, the footswitch DC isolation barrier circuit 258, and safety interlock switches (not shown here) e.g. to indicate that a panel or cover has been removed and to disable the system accordingly. Signals sent from microprocessor unit and signal conditioning circuits 214 include: the TTL control signal 205 for the PIN diode modulation switch 204, the digital control lines 209 to the PIN diode attenuator 206, and information signals to the user control and display unit 218,220.

The microprocessor performs various mathematical functions, such as the calculation of energy delivered into the biological tissue load 230, and interprets control information sent from the user control and display unit 218,220. The microprocessor may be a 16, 32 or 64 bit unit and may include a digital signal processor (DSP) board. The user control and display unit 218,220 enables the user to specify engineering mode functions, such as RMS and/or peak power level, duty cycle, pulse width, pulse shape and user defined pulse sequences, i.e. swept variation of pulse amplitude and/or pulse width over a predetermined period of time. The user control and display unit 218,220 may also be configured to only operate in surgical mode, whereby user specified functions and displayed information is limited, for example, it may be only be necessary to input lesion size or dose of energy delivered into tissue, and the output display may be simply a bar graph showing energy level and remaining treatment time. It may be desirable for the user/operator to specify the treatment modality only (e.g. GORD) whereby the system is arranged to send out the required energy profile (e.g. dose and pulse pattern) to treat this condition.

It is also necessary for the system to monitor for fault conditions. For example, reflected power detector 213 is used to establish that the cable assembly is connected properly and that the antenna is functioning correctly. Demand power and actual delivered power, monitored by detector 211, are compared to ensure that the output power is within a specified range of the demand power level. The microprocessor 214 is used to monitor the fault conditions and will flag up any error that is detected and may be arranged to switch off the system depending on the severity of the fault.

The apparatus may also include a supervisory channel and a means of cross checking communication between the microprocessor and said supervisory channel. This is to ensure that the data sent out by the microprocessor is correct and that the microprocessor is working correctly. This is a method of ensuring that, for example, 50 W does not appear at the output of the amplifier 208 when a 1 W demand has been selected. The supervisory channel may be an additional microprocessor, a single bound computer or even program running on the same microprocessor used to perform checking. The supervisory channel is often referred to as a "watchdog".

The user control and display unit 218,220 includes a touch screen module, a LED/LCD display 220 and switches contained e.g. within a waterproof membrane, or any other suitable input/output device.

The initiation of energy delivery is via footswitch pedal 222. A footswitch DC isolation barrier circuit 258 is connected between said footswitch pedal 222 and the microprocessor unit and signal conditioning circuits 214 to provide a DC isolation barrier between the footswitch circuit (connected to the user via an electrical cable), and the microwave radiation generating line up (consisting of the components described above).

For implementation of the power control scheme, a power demand may be set-up using the microprocessor unit 214 in accordance with the energy dosage, lesion size lesion type, or treatment modality instructions input by the user via the user control and display unit 218,220. The microprocessor unit 214 will send out a digital control signal 209 to PIN diode attenuator 206, and the information received by forward power detector 211 will be compared with the power demand signal and the difference will provide an error voltage that will be used to adjust (increase or decrease) the digital signal 209 applied to the PIN diode attenuator 206 in order to force the output power level to follow the demanded power level. It is also possible to take into account the signal from the reflected power detector 213 when developing the power control algorithm. In this instance, the difference between the forward power measurement and the reflected power measurement would be compared with the demand signal and adjustments would be made to enable the net power delivered into the biological tissue 230 to be the same as the demanded power level.

A power source unit 240, e.g. battery, or AC to DC converter provides operating power (e.g. DC current) to the devices in the apparatus. In the arrangement shown in Fig. 3, the power source unit 240 has eight outputs $V_1$ to $V_8$. $V_1$ powers the microwave source 200, $V_2$ powers the PIN diode switch 204, $V_3$ powers the PIN attenuator 206, $V_4$ powers the preamplifier 207, $V_5$ powers the power amplifier 208, $V_6$ powers the microprocessor 214, $V_7$ powers the user control and display unit 218,220, and $V_8$ powers the footswitch DC isolation circuit 258.

Treatment Probe

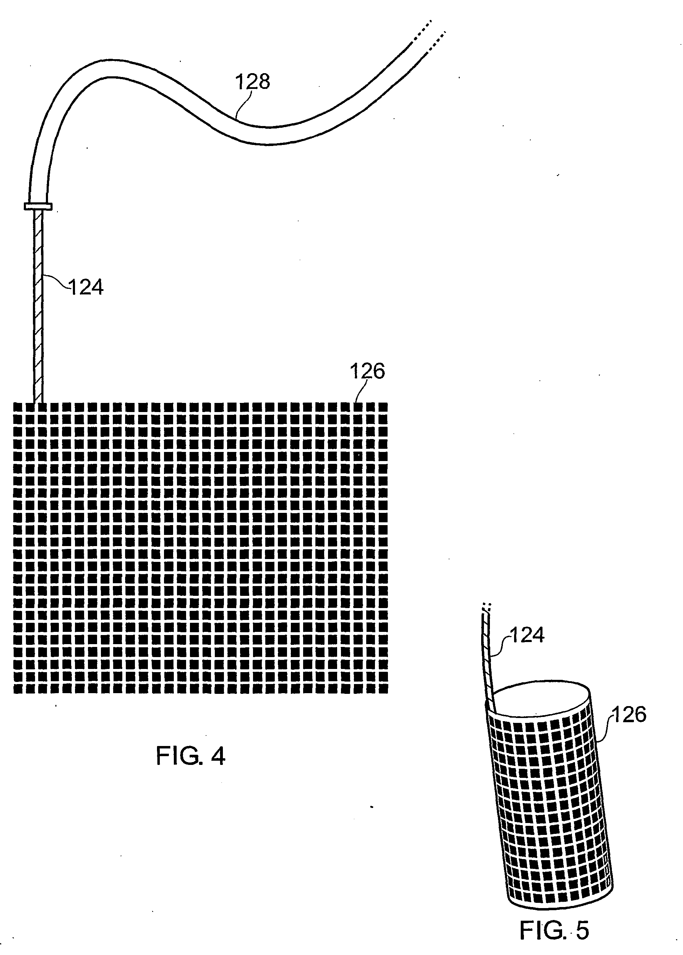

The configuration of the treatment probe is now discussed with reference to Figs. 4 to 21. Fig. 4 shows one arrangement of the basic three-part construction at the treatment end of the apparatus shown in Fig. 3. A microwave signal is provided from the microwave radiation generating line up through co-axial cable 128. This power is transferred into a thinner co-axial feed cable 124 which is arranged to supply the power to an array of patch antennas 126. The array of patch antennas 126 is formed on a flat sheet of material, which is preferably flexible. The microwave power supply from the feed cable 124 is divided in order to provide substantially equal power to each patch antenna, which is configured to radiate an electromagnetic field at a frequency that lies within the microwave frequency region of the electromagnetic spectrum.

The array 126 and feed cable 124 are adapted to be inserted into the oesophagus. This adaptation may involve coating them in a biocompatible material (e.g. Parylene C) and rolling the sheet of patch antennas into a tube as shown in Fig. 5, where the radiating patch antennas are facing outward (i.e. towards the oesophageal wall during treatment). As mentioned above, the feed cable 124 is semi rigid to facilitate the ability to push it into position. It is preferable for feed cable 124 to be a flexible semi-rigid construction.

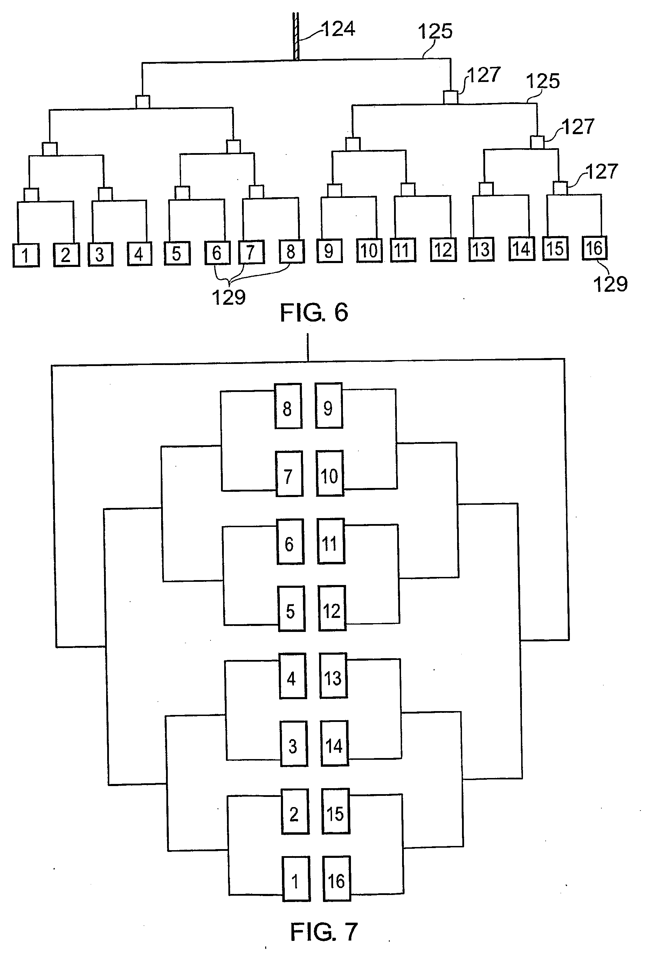

Figs. 4 and 5 show a 2-D patch antenna array structure. However, it is possible to have only a 1-D array, e.g. if treatment with very fine precision is required. Thus, the tube as shown in Fig. 5 may only have a single ring of patch antennas around its circumference. The single ring may, for example, take the form of a travelling wave linear array or a linear microstrip patch array with a series feed configuration. Fig. 6 shows an example of a power feed structure for 16 patch antennas arranged in a 1-D array. The feed structure has a cascading tree-like configuration comprising a series of T junctions where an input line splits into two output lines. Thus, the supply from the feed cable 124 initially splits into two branches, each of which is split into two further branches, and so on until there are sixteen branches, each of which feeds a single radiating patch antenna. To improve the power transfer through the system (i.e. to prevent the signal from being reflected) the impedance of each input branch is matched to the impedance of the two output branches. In Fig. 6 this is achieved by providing a quarter wave transformer 127 at the junction where an input transmission line 125 splits into two output transmission lines. In the case where the impedance of the input and output transmission lines is different, the impedance of the quarter wave transformer 127 needs to be calculated to ensure proper impedance matching. If the input impedance is denoted as $Z_i$ the output impedance as $Z_o$, then a line of length equal to a quarter wavelength (or an odd multiple of quarter wavelengths) and of an impedance equal to $\sqrt{(Z_i Z_o)}$ may be inserted between the two impedances to perform the impedance matching function. An alternative configuration is possible if transmission lines with the same impedance are used throughout the feed structure, for example, the complete feeding structure may use 50Ω lines. In this case, separate quarter wave transformers are not required. Instead, alternate branches of the feed structure may be constructed with a length equal to an odd number of quarter wavelengths (i.e. $(2n-1)\lambda/4$). These branches act as effective quarter wave transformers. The intervening branches (the transmission lines in between the alternate quarter wavelength lines) can be of any length as long as the distance of all output lines from a given junction to the next junction is equal.

Fig. 7 shows a 2-D arrangement with a parallel connection feed structure similar to Fig. 6. The networks in Figs. 6 and 7 are known as corporate fed networks.

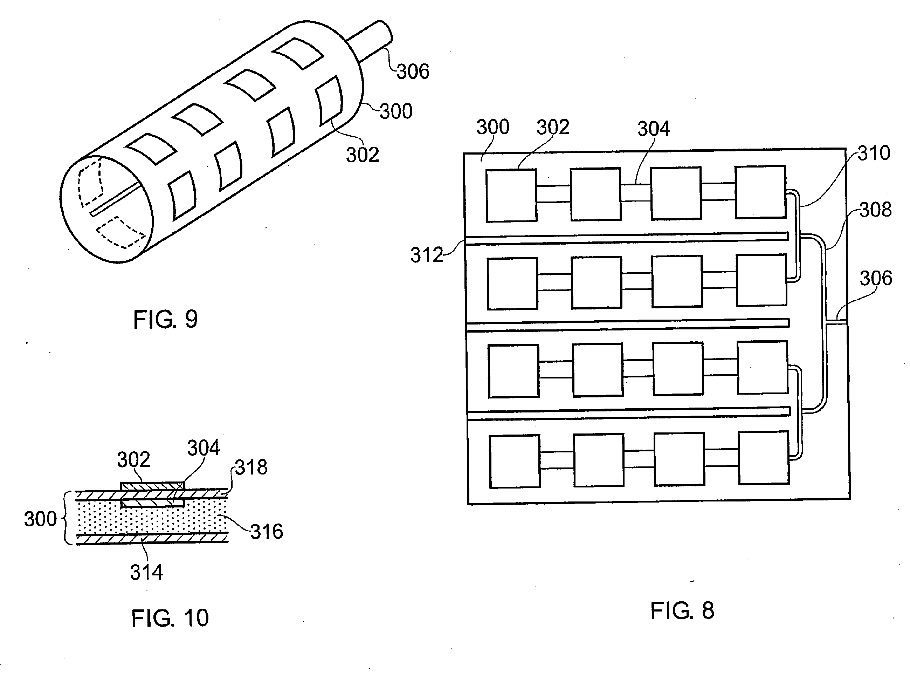

Fig. 8 shows an alternative 2-D arrangement. For convenience, Fig. 8 shows the array in a flat configuration. A substrate 300 has sixteen patch antennas 302 mounted on it in four rows of four, where each row of four is connected in series by a transmission line 304. Power is supplied to each of these rows from a main power line 306 which is split into parallel branches 308, 310 in a way similar to that shown in Figs. 6 and 7 (i.e. all junctions are impedance matched). Slots 312 are formed in between each adjacent row of antennas 302. The slots 312 are shown as continuous, but they may be separated into a plurality of discrete slots. The substrate 300 is formed of a flexible material (e.g. Rogers R/flex 3000 or flexible substrate and foils from Sheldahl Technical Materials (www.sheldahl.com)), which enables it to be rolled into a tubular form suitable for insertion into the oesophagus as shown in Fig. 9. Fig. 9 shows the feed cable 306 passing through the centre of the tube before looping back to attach to the substrate 300.

It is desirable to prevent coupling between any electromagnetic field produced by the transmission lines 304 between adjacent patch antennas 302 and the tissue being treated. Ideally, the treatment field should be produced by the patch antennas 302 alone. To achieve this, the connecting transmission lines 304 are shielded from the tissue by an extra layer of dielectric material. This structure is shown in detail in Fig. 10. Here it can be seen that the substrate comprises a base conducting layer 314 having a first dielectric layer 316 mounted on it. The transmission lines 304 that interconnect the patch antennas 302 are mounted on this first dielectric layer 316, and then have a second dielectric layer 318 deposited thereon. The patch antennas 302 are mounted on the second dielectric layer 318. Connections (not shown) are provided to ensure that the transmission lines 304 can transfer the desired power to the patch antenna 302. The relative permittivity of the second substrate 318 may be higher than that of the first substrate 316 to provide high coupling to the radiating antenna. The thickness of second substrate 318 may be less than that of the first substrate 316 in order to couple the desired amount of energy between the two.

Although the arrangement shown in Figs. 8 and 9 has radiating patches 302 located all the way round the circumference of the tubular substrate 300, this is not an essential feature of the present invention. If desired, patch antennas 302 may extend only partly (e.g. 90° or 180°) around the circumference of the tube. This can allow the probe to treat only one side of the oesophagus. In some instances, it may be desirable to use a single radiating patch or a line of patches or slots to form a "paddle" and then physically move or manipulate the "paddle" to treat the circumferential lesion. The probe can be moved around mechanically within the oesophagus, e.g. by manipulating the semi-rigid cable, thereby allowing different portions of the oesophageal wall to be treated.

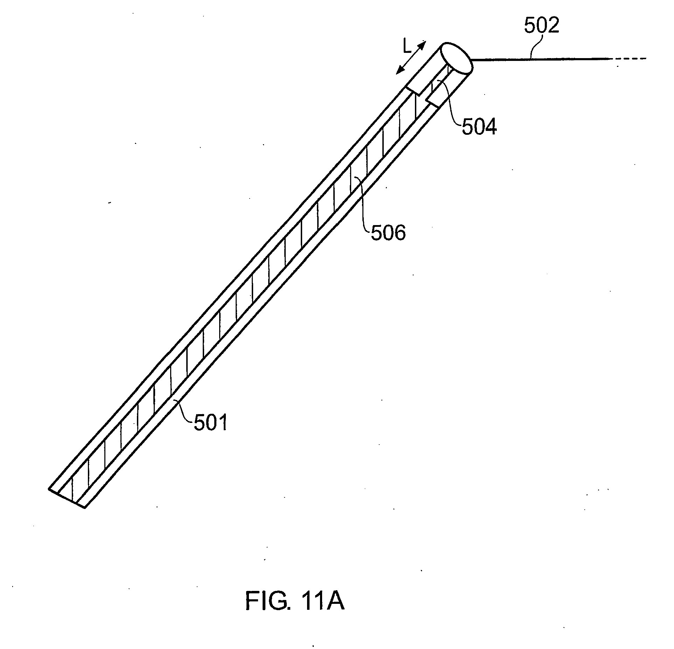

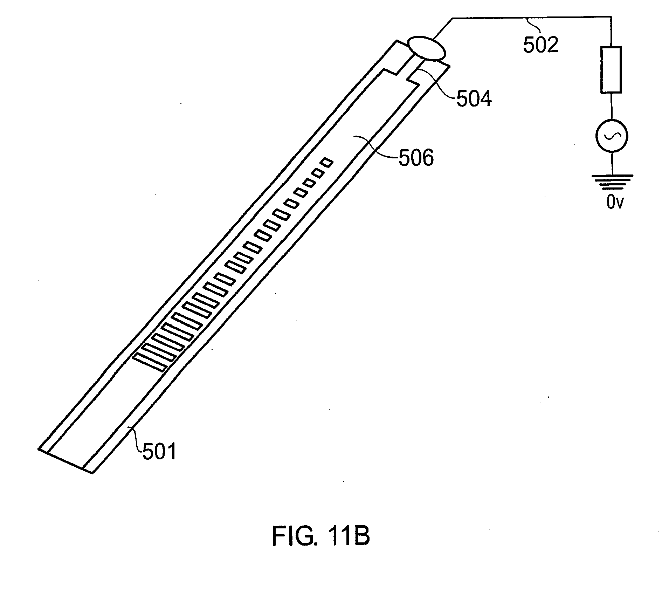

Figs. 11A and 11B show a flat (before rolling) substrate with an alternative radiating structure. The substrate has a conducting ground plate that is separated from a thinner upper conducting plate by a dielectric layer. In Figs. 11A and 11B, instead of forming the upper conducting element into a plurality of patch antennas, a series of slots are formed in the upper conducting layer, and it is from these slots that an electromagnetic field radiates. The slots are positioned so that the maximum electric field is emitted from them in practice, this means they may be separated by $\lambda/2$, where $\lambda$ is the guided wavelength, which is a function of the relative permittivity of the substrate material, and, when the antenna is in contact with the tissue to be treated, the relative permittivity of the tissue. $\lambda$ is a function of the inverse of the square root of the relative permittivity of the substrate and the biological tissue; thus materials with a higher value of relative permittivity can lead to more closely spaced slots, which may result in more uniform thermal damage. $\lambda$ is also a function of the microwave frequency.

Thus, the structure shown in Fig. 11A consists of a 50 Ω feed line 502, followed by a quarter wave impedance transformer 504 having a length (L) of 3.2 mm and characteristic impedance of 25 Ω. L is determined by the relative permittivity of the substrate material and the microwave frequency.

This transformer enables the 50 Ω feed line 502 to be impedance matched to the impedance of a slotted radiator 506 (the slots of which are not shown in Fig. 11A), which has a characteristic impedance of 12.5 Ω (i.e.

$$\sqrt{50 \times 12.5} = 25\,\Omega).$$

Fig. 11B shows the radiating slots on the slotted radiator 506. In this embodiment, the slot width is changed along the radiator to ensure that the energy transferred into the biological tissue is uniform along the radiator 506. It can be seen that the slot closest to the generator has the shortest length and the slot furthest away from the generator has the longest length. In this way, the same amount of energy emanates from each slot to enable the biological tissue to be exposed to the same amount of energy along the length of the antenna. In the particular embodiment shown in Fig. 11B the spacing between the slots is 0.6 mm and the width of the slots is 0.4 mm, thus the pitch is 1 mm. The structure consisted of 20 slots and the overall length of the structure shown is 19.4 mm.

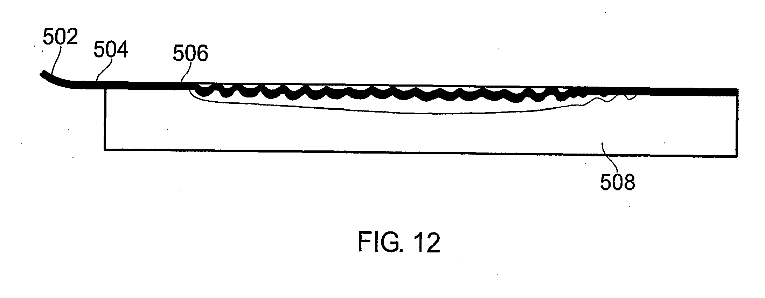

The energy distribution into the oesophagus at 14.5 GHz is shown in Fig. 12. The power density (W/m$^2$) delivered into tissue 508 is essentially uniform over the length of the radiating structure, thus by designing a structure with correct slot width, length and spacing a practical antenna can be realised.

In the structures shown in Figs. 11A and 11B and simulated in Fig. 12, the width of the radiating slot line was 2.5 mm, thus the structure could be inserted down the instrument channel of an endoscope and used as a "paddle" construction for treating the walls of the oesophagus. A paddle structure similar to one shown here has been identified as a useful surgical tool for the treatment of oesophageal disorders.

If the length of the paddle (slot radiator) was required to be extended then this would be a relatively straightforward process, i.e. variation of the geometry of the radiating slots could enable a structure to be developed with an overall length of more than 40 mm. The width of the widest slot would be similar to the width slot shown in Fig. 11B, hence a 40mm radiating structure could be fitted inside the 2.8mm diameter instrument channel of the standard endoscope.

In the structure show here the ground plane covers the full width of the dielectric, i.e. 2.5 mm. The thickness of copper for the top and bottom metallisation was 0.018 mm and the thickness of the dielectric layer was 0.1 mm. These dimensions correspond to the Rogers R03003 or R03006 flexible laminate materials.

The slotted structure effectively produces a set of individual field distributions. To achieve a uniform tissue effect, it is desirable for these individual field distributions to overlap with one another, as shown at the right hand end of Fig. 12. By combining a series of effectively out of phase field distributions, the overall uniformity along the length of the array (around the circumference of the probe) can be improved.

In an alternative configuration, the slot-type probe can be constructed from a co-axial tube structure having an inner conducting element separated from an outer conducting layer by a dielectric material. Circumferential slots may be formed in the outer conducting layer, i.e. the dielectric layer may be exposed at intervals along the length of the tube. As above, the slots need not extend fully around the circumference of the tube and are separated by λ/2, with the first slot located at a distance of λ/4 away from the distal end of the tube and the distal end is terminated with a good short circuit in order that the maximum electromagnetic field is radiated at a distance of λ/4 and odd multiples thereof from said short circuit.

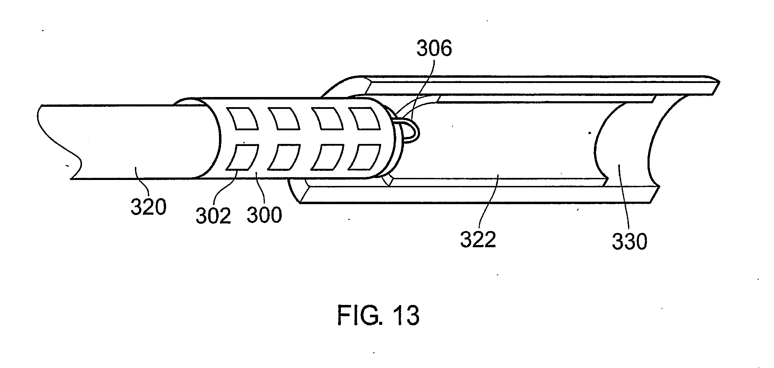

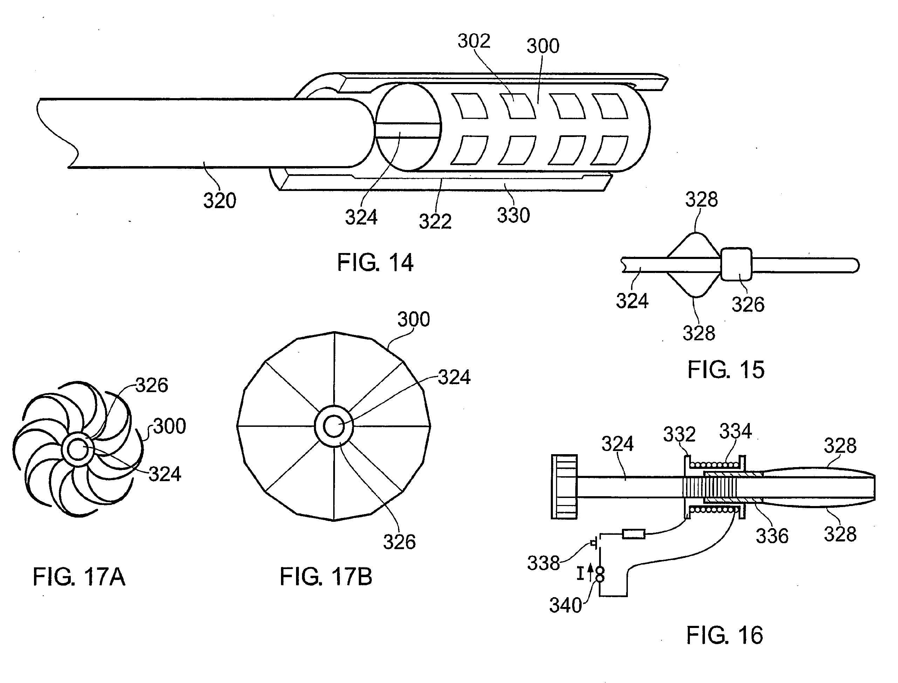

Figs. 13 and 14 depict insertion of the probe into an oesophagus and treatment of the oesophagus respectively. In Fig. 13, the tubular probe 300 shown in Fig. 9 is slid around the circumference of an endoscopic tube 320. The tube is then inserted e.g. through the nose cavity to the oesophagus 330 in order to ablate a treatment area 322. During insertion, the substrate lies in a compact access configuration against the surface of the endoscopic tube 320. Once in position, the probe 300 is pushed away from the end of the endoscopic tube 320 by a carrier rod 324 (which may be the semi-rigid feed cable described above). An open/close mechanism (not shown) is mounted on the carrier rod 324 and, when activated, causes the probe 300 to expand as shown in Fig. 14 so that the radiating patch antennas are brought into close proximity or contact with the treatment region 322.

Fig. 15 shows the open/close mechanism on the end of carrier rod 324 with the probe 300 removed. The open/close mechanism includes a axially moving member 326 mounted on the rod having an electrical connection (not shown) that extends back through the rod so that the user is able to control the movement of the element 326 from outside the device. Spring members 328 have one end attached to the carrier rod 324 and another end attached to the movable element 326 so that, when the movable element 326 moves along the rod 324, the springs 328 extend or retract out of or into the rod 324. When the probe 300 is positioned over this mechanism, the springs 328 are arranged to push it outward e.g. towards the treatment region.

Fig. 16 shows the operation of the open/close mechanism in more detail. The axially moving element 326 comprises a housing 332 fixed in position on the rod 324. The housing holds a coil 334 through which current flows from current source 340 when switch 338 is depressed. A sleeve 336 is slidably mounted on the carrier rod 324 underneath the coil 334. The sleeve is manufactured from a material having a high relative permeability (higher than the relative permeability of the carrier rod) so that when current flows through the coil 334 a force experienced by the sleeve 336 due to the magnetic field generated by the current flow in the coil 334 causes the sleeve to axially move along the rod 324. The springs 328 are fixed to the rod 324 and the sleeve 336.

The probe 300 may be carried inside the endoscopic tube to the treatment region. Figs. 17a and 17b show a cross-section through the probe 300 demonstrating respectively an access configuration where the diameter of the probe 300 is small enough to fit inside the endoscopic tube 320 and a treatment configuration where the probe 300 has expanded to bring the patch antennas 302 into close proximity with the treatment region. The probe 300 may be attached to the springs 328 so that it moves with them. Alternatively, the probe 300 may be at rest in or naturally biased towards the access configuration, e.g. the folded configuration shown in Fig. 17A, so that when the springs are retracted, it automatically shrinks away from the expanded configuration shown in Fig. 17B.

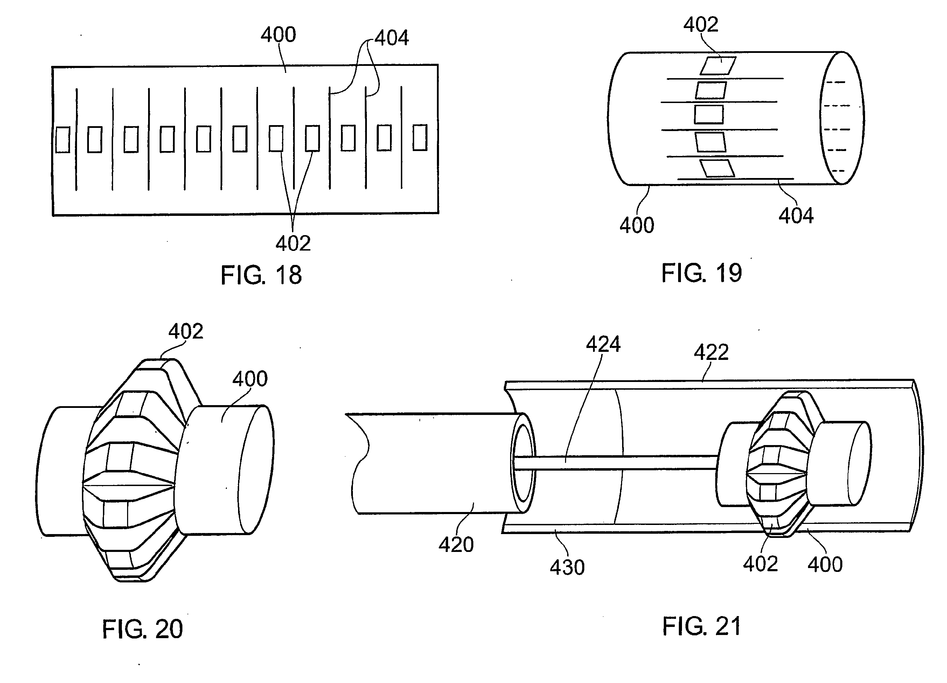

Another embodiment of the probe is shown in Figs. 18 to 21. As with earlier embodiments, the probe is formed from a flat substrate 400, which has patch antenna structures 402 fabricated on to it. In this embodiment, each patch antenna is separated by a slit 404 through the substrate 400. A single, or a plurality of radiating slots can be used to form the lantern arrangement. In order to get a uniform energy profile from a slotted radiator the sizes of the slots are varied along the length of the slotted radiator in order for the energy or fields emitted from each slot to be the same, so as to produce a uniform energy profile inside treatment tissue and produce uniform thermal heating of the tissue. As shown in Fig. 19, the substrate is rolled into a tubular form for use such that the slits 404 are parallel to the axis of the tube. In this configuration, when the ends of the tube are pushed towards one another, the strips of material that lie between the slits 404 bulge outwards, as shown in Fig. 20. By placing the patch antennas 402 in the middle of the strips, the bulging causes the antennas to extend outwardly, e.g. to be brought into close proximity or contact with the treatment region. Fig. 21 shows this configuration in use. Initially, the probe 400 is carried to the treatment site in an endoscopic tube 420. Once near the treatment location 422 in the oesophagus 430, the probe 400 is pushed out of the end of endoscopic tube 420 using the carrier rod 424 ( flexible semi-rigid feed cable). The lantern construction may be twisted in order to reduce the separation between radiating patches. This may help to make the field more uniform. In this instance, the array of patches will need to be longer to accommodate the twist. An axially moving open/close mechanism is mounted on the carried rod 24 and attached to one end of the probe 400. The other end of the probe is attached to the carrier rod 424 such that, when the open/close mechanism moves along the rod, the ends of the probe 400 are moved towards or away from one another to cause the strips carrying the patch antenna 402 to expand or retract. When the probe is moved into its treatment (expanded) configuration, power is supplied to the patch antennas 402 which radiate an electromagnetic field which penetrates the wall of the oesophagus 430 to a controlled depth or wall thickness.

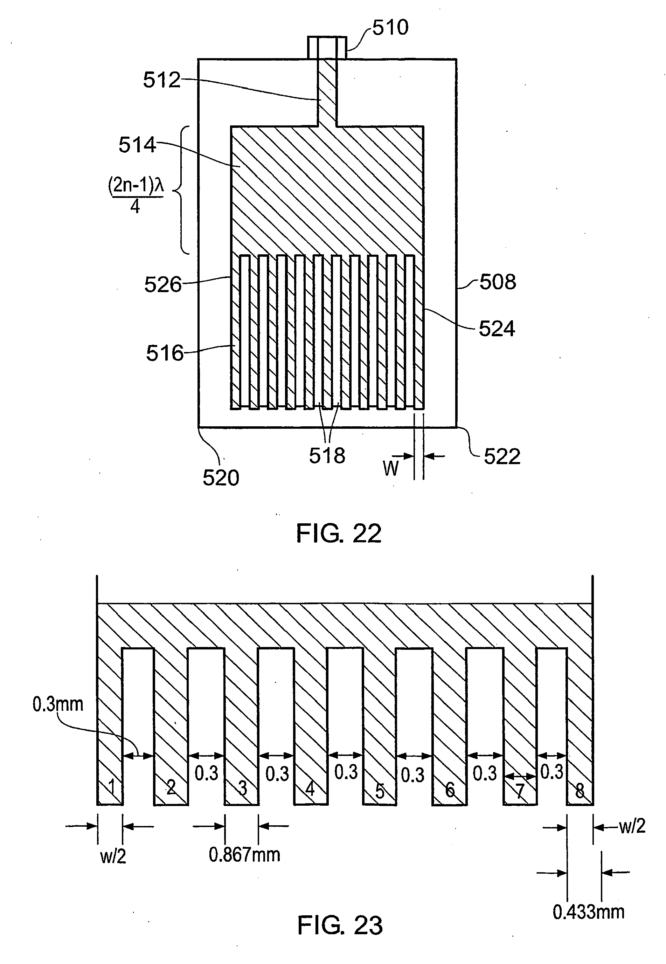

Fig. 22 shows a configuration where a plurality of microstrip lines 516 are used instead of patch antennas. In this configuration, the lines form a tube and can be compressed (as in the Chinese Lantern) and/or twisted to enable the radiating lines 516 to make greater contact with the walls of the oesophagus.

The microstrip lines are fabricated on to a flexible substrate material 508 with a metallic coating on each side. Fig. 22 shows a single impedance transformer 514 connected between the 50 Ω feed line 512 and the microstrip lines 516. The matching transformer 514 is a line of length equal to an odd multiple of the quarter wavelength at the frequency of interest (i.e. $\dfrac{(2n-1)\lambda_L}{4}$ where n is an integer and $\lambda_L$ is the loaded wavelength). The impedance ($Z_t$) of the line that constitutes the transformer is selected to be equal to the square root of the source impedance ($Z_s$), which is 50 Ω here, multiplied by the impedance of the microstrip lines ($Z_m$) divided by the number of lines in the structure i.e.

$$Z_t = \frac{\sqrt{(Z_s Z_m)}}{n},$$

where $Z_t$ is the characteristics impedance of the transformer, $Z_s$ is the source impedance, $Z_m$ is the impedance of each of the n microstrip lines, and n is the number of lines. It may be more appropriate to have the matching transformer positioned at the proximal end of the feed cable assembly 224. In this instance, the characteristic impedance of cable 224 maybe adjusted to match the antenna feed directly with the cable and a transformer between the proximal end of the cable 224 and the 50Ω generator may be used.

In Fig. 22 there are ten microstrip lines 516, each separated by a slot 518 which is a cut out of both the metal layer and the substrate layer.

Practically, substrate edge 520 is then joined to substrate edge 522 by sealing or otherwise to form a tubular structure. The width of the endmost radiating elements 524, 526 can be made to be half the width of the intermediate elements so that the outmost edges of end elements 524, 526 may be joined together e.g. by soldering. In this instance, substrate material 508 will be cut away to expose the outmost edges of end elements 524, 526. The slots 518 between the radiating lines are cut out to enable the structure to be twisted and compressed. In the structure shown in Fig. 22 there are 9 slots 518 cut for the 10 radiating microstrip lines 516.

The feed transformer 514 is not limited to using a $\dfrac{(2n-1)\lambda_L}{4}$ microstrip line. Other feed configurations that have already been addressed in this description may be used. The advantage of using the (single transformer) feed structure is that the construction is simple and radiation from the transformer 514 itself us limited due to it being made from a single line. When a large impedance transformation is required, i.e. it is required to match a 1Ω load to a 50Ω source, it may be appropriate to use two λ/4 transformer sections. For example, the first section may be 20Ω and the second section may be 141Ω.

It is desirable to coat conducting surfaces (metallised patches or radiators) with Parylene C or other suitable biocompatible material. Parylene C is preferred due to the fact that it is biocompatible, easy to apply as can be conformably coated to the structure. Considering that a typical instrument channel contained within an endoscope is 2.8 mm in diameter and assuming that 2.6 mm of that is usable, then the circumference of a rolled patch array of the microstrip structure is πd = 8.17 mm.

Thus, if one wrap (or a single wind) is used, i.e. the structure has no overlapping areas, the seven strip structure with dimensions as shown in Fig. 23 can be used.

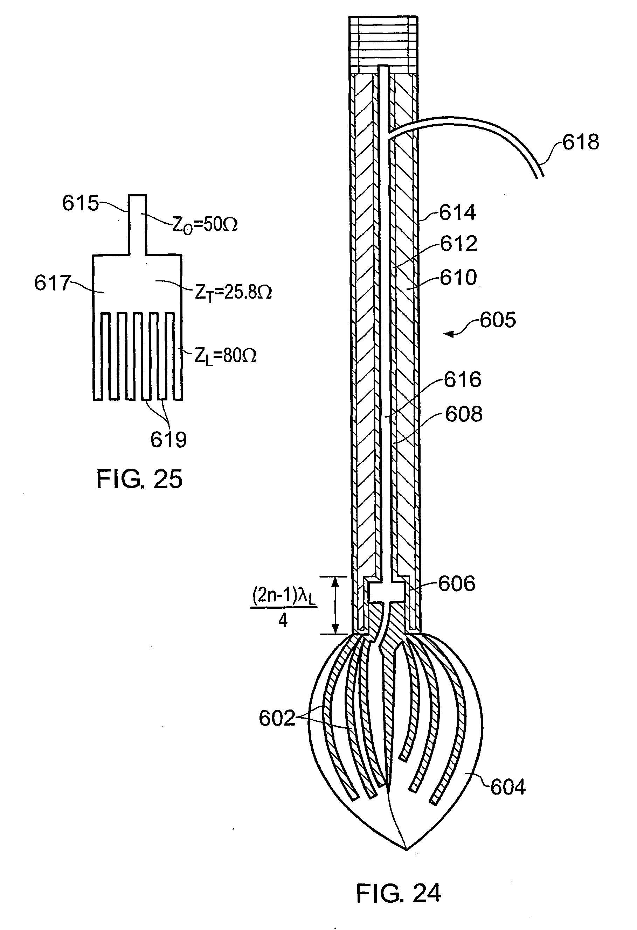

Fig. 24 shows a further embodiment, in which a plurality of microstrip lines 602 are attached to a surgical balloon 604 and fed from a single quarter wave transformer 606 attached to a single feed line 608 (e.g. having a characteristic impedance of 50 Ω, but not limited to this value), where the feed line forms a part of the inflation (e.g. water delivery) system.

In this aspect of the invention the theory that microwave energy is confined to very thin layers of metallisation is used to enable the centre conductor of the co-axial structure to be hollow and allow water to be pumped into the balloon without affecting the microwave properties of the structure or the propagation of the electromagnetic field.

There are a number of advantages with using this structure. Firstly, the 2.8 mm diameter endoscope channel can be used to propagate enough microwave energy to cause controlled ablation of the oesophagus with an appropriate antenna probe structure fabricated into e.g. a conventional water inflatable medical balloon.

Water filled balloons are preferred to air filled balloons for the reason that the pressure required to inflate is less, hence should the balloon burst inside the patient then less damage will be caused by the water filled structure. A further advantage is that the water in the centre conductor may help to keep the microwave feed structure cool even in the event whereby high power levels (preferably pulsed) are applied.

As shown in Fig. 24, a practical embodiment of the microwave feed line/water channel 605 is that of using a dielectric rod 610 (for, example nylon or PET) and removing the centre of this rod to form a thick walled tube. The inner wall of the tube has a metallisation layer 612 formed thereon, made from a good conductor with a thickness selected to allow substantially all of the microwave energy to propagate. The outer wall of the tube also has a metallisation layer 614 to provide the second conductor (e.g. grounded return conductor) of the probe. The combination of inner metallisation layer 612, dielectric wall 610, and outer metallisation layer 614 forms a co-axial structure which allows the microwave energy to propagate whilst also providing a centre channel 616 to allow water to be pumped in and out of the system from source 618 to allow the balloon to be expanded and collapsed (inflated and deflated). The source 618 may be a syringe with a pressure gauge, for example.

This arrangement lends itself particularly well to the preferred frequency of operation, i.e. 14.5 GH$_z$ as the energy is confined to a very thin layer of metallisation. Table 1 below shows the results from calculations for the thickness of common conductive materials that may be considered.

Table 1: Percentage of microwave energy and thickness for common conductors at a frequency of 14.5 GHz

| Material | Thickness (μm) | | |
|----------|------|------|--------|
|          | 90%E | 99%E | 99.9%E |
| Copper   | 1.26 | 2.53 | 3.79   |
| Silver   | 1.23 | 2.46 | 3.68   |
| Nickel   | 2.69 | 5.38 | 8.07   |
| Steel    | 4.3  | 8.61 | 12.91  |

From these results, it can be seen that the thickness required for the poorest conductor to ensure that substantially all of the energy flows is 12.91 μm = 0.0129 mm.

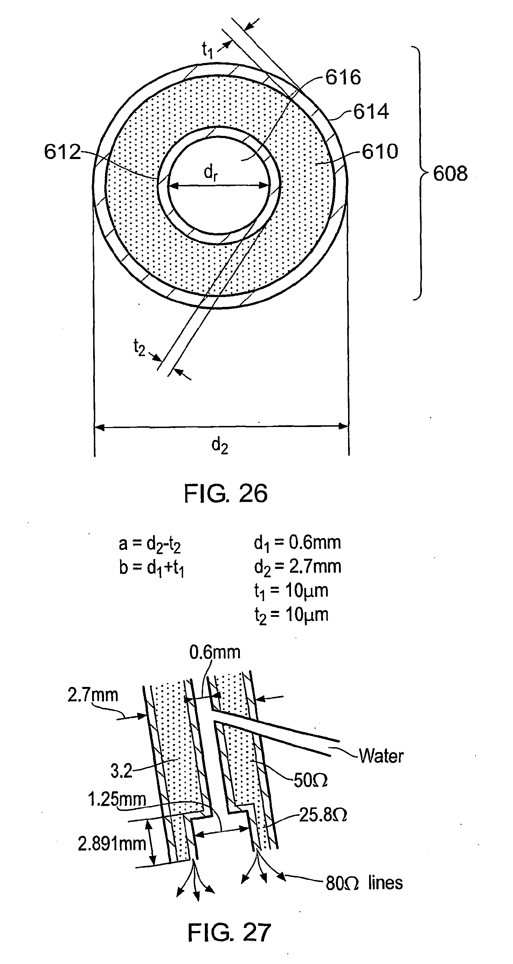

If we assume that the relative permittivity of nylon at 14.5 GHz is 3.2 and that a 50 Ω feed line is required. Also assuming that the an instrument of outer diameter of 2.7 mm can be fitted down a standard endoscope instrument channel with a diameter of 2.8 mm, then the diameter of the tube to feed water through is approximately 0.6 mm, as demonstrated by the following equation and illustrated in Fig. 26. The characteristic impedance of a co-axial structure ($Z_o$) is given by

$$Z_o = \frac{138}{\sqrt{\varepsilon_r}} \log_{10} \frac{a}{b},$$

where $\varepsilon_r$ is the relative permittivity of the rod (tube), a is the inner diameter of the outer conductor, and b is the outer diameter of the inner conductor.

Fig. 25 shows a schematic representation of the probe structure illustrated in Fig. 24. It has three parts: the feed line 615, the quarter wave transformer 617 and the microstrip (radiating) lines 619. There are six microstrip lines 619, which (as shown by lines 602 in Fig. 24) are attached to the surface of the balloon 604. The feed line 615 and transformer 617 are formed by the co-axial tube structure described above. The inner diameter of the co-axial tube at the transformer section is selected to give the required impedance to make the desired impedance transformation, i.e. to match the feed line with the microstrip lines 61a.

If the feed line 615 has an impedance of 50 Ω and the six radiating microstrip lines (or wires) 619 each have impedance of 80 Ω, the co-axial quarter wave transformer 617 needs an impedance of $\sqrt{\left(\frac{80}{6}\right) \times 50} = 25.8\,\Omega$ to match the feed line 615 to the six radiating lines 617. In order to realise this impedance within the structure using the nylon rod described above, the diameter of the centre conductor will need to be modified to

$$\frac{2.7}{10 \times \frac{25.8 \times \sqrt{3.2}}{138}} = 1.25\,\text{mm}.$$

Therefore, the diameter of the hole through the rod will be increased for a length equal to an odd multiple of a quarter of the loaded wavelength (or $\frac{(2n-1)}{4}\lambda_L$, where $\lambda_L$ is the loaded wavelength and n is any integer). In this instance the length could be $\frac{3 \times 10^8}{14 \times 10^9 \times 4 \times \sqrt{3.2}} = 2.891\,\text{mm}$. If this length is inconvenient, then 8.674 mm or 14.455 mm may be used i.e. $\frac{3\lambda_L}{4}$ or $\frac{5\lambda_L}{4}$. A magnified view with dimensions of this structure is shown in Fig. 27.

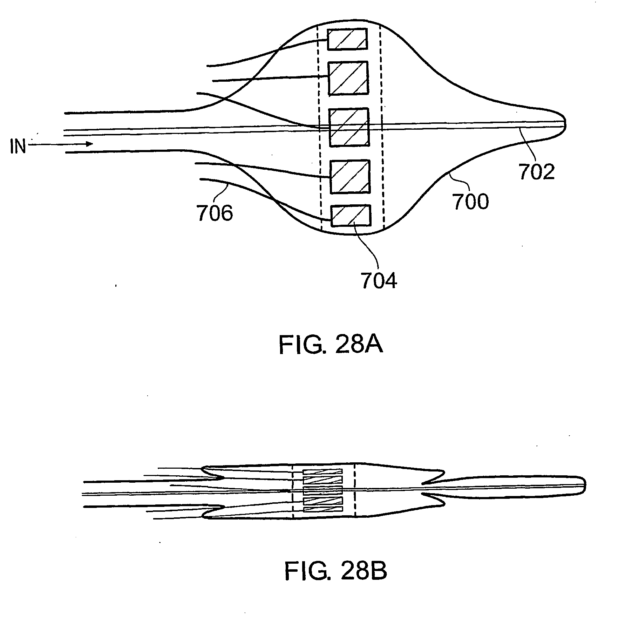

Figs. 28A and 28B illustrate a conical balloon 700 in an inflated and collapsed state respectively. In this case, the radiating elements are conducting patches 704 fabricated around an expandable circumference of the surface of the balloon 700. A rigid guide rod 702 (e.g. of stiff wire and often referred to as a catheter) attached within the balloon 700 allows the user/operator control over the balloon's position. Air or water are introduced at a proximal end (marked IN on Fig. 28A) to inflate the balloon 700. Each conducting patch 704 is fed by a co-axial cable 706 e.g. of the type described above. It may be preferable to feed each co-axial cable up the instrument channel of the endoscope and combine the cables at a location outside the endoscope. Power combining could be achieved using a quarter wave transformer, a microstrip combiner or a waveguide combines.

Medical balloons are normally made from flexible polyvinyl chloride (PVC), cross linked polyethylene or polyethylene terephthalate (PET). Other materials include nylon and polyurethane. The balloon itself may be wrapped around a catheter shaft to minimise its profile. A vacuum may be pulled through the balloon to collapse it.

In fact, an inflatable balloon may be used as a carrier for different types of radiating structures. For example, the conducting patch array, the slot line antenna, the coplanar waveguide and the radiating monopole structures discussed above may all be mounted on a surgical balloon to gain access to the treatment site.

To distribute plural radiating structures (e.g. slot line antennas) around the balloon circumference in a balanced equidistant manner, a half wave transformer may be used between the feed line and the radiating structures. As explained above, transmission line theory provides that transformers or transmission lines with a length that is an odd multiple of half wavelengths are (assuming a low loss line) transparent in terms of impedance. Thus, in theory a half wave transformer can be made with any width without affecting the microwave environment.

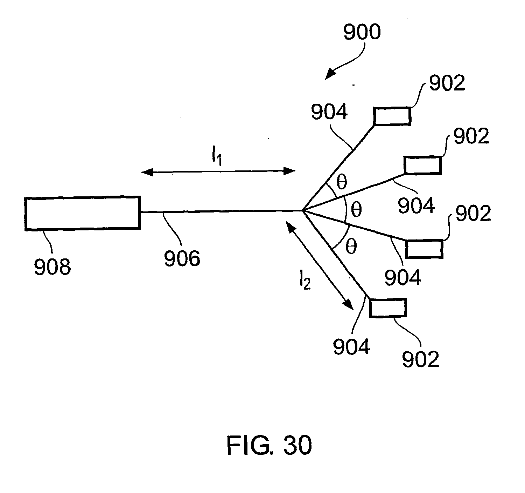

In another embodiment, the feed structure may include a split from a single point on a primary feed line to a plurality of secondary feed lines. The secondary feed lines can be angled with respect to each other to enable the antennas that they feed to be distributed over the balloon surface. Fig. 30 shows a schematic illustration of an example of such a feed structure. Fig. 30 illustrates a feed structure 900 which provides power from a single 50 Ω cable 908 (e.g. having a structure as discussed above) to four antenna feed lines 902 which feed four radiating slot antennas (not shown) mounted on a surgical balloon (also not shown). The feed structure is configured to distribute the antennas equally over the balloon surface yet achieve a substantially equal division of power between them. A primary feed line 906 having a predetermined length $l_1$ splits at a single point into four secondary feed lines 904, which are at a predetermined angle θ with respect to each other and each of which have a predetermined length $l_2$. The length of the primary and secondary lines 904, 906 depend at least in part on their respective impedances. In a practical embodiment where both the primary and secondary lines are 25 Ω lines and the antenna feed lines each have an impedance of 12.5 Ω, selecting value of $l_1$ and $l_2$ as 3.2 or 9.6 mm and the value of θ to be 40° yields a substantially equal power division between the antennas. Any of the secondary feed lines 904 can be curved gently, to allow the 25 Ω lines to curve up four sides of the balloon after the split point. The lengths given are to be measured along the centre of the feed lines, and the centres of the secondary feed lines 904 meet at the centre of the end of the primary feed line 906, which where the lengths $l_1$ and $l_2$ are measured from. If any odd lengths need to be added to get the four antennas in line with each other, this can be done using the 12.5 Ω antenna feed lines 902 without affecting the split structure.

The balloon itself can be a dielectric material on to which conducting material (e.g. metal foil or the like) is deposited to form the radiating structures. Alternatively, the balloon may be all or part metal e.g. so that its surface can act as a slot line radiator.

In another embodiment, the balloon may have two layers: an outer conducting (e.g. metal foil) layer and an inner dielectric layer (e.g. PVC, PET or the like). Holes formed in the dielectric layer will then act as radiating patches.

**Claims**

1. A probe (226) having an access configuration in which it is insertable into an oesophagus and a treatment configuration in which it is operable to treat an area of oesophageal wall tissue with microwave radiation, the probe having:

   a flexible substrate (300);
   one or more radiating elements on the flexible substrate;
   a feed structure (224) arranged to energise the radiating element(s), thereby causing the radiating element(s) to emit microwave radiation; and
   deployment means (326) arranged to transfer the probe from its access configuration to its treatment configuration,
   the feed structure (224) includes a grounded conductor (314) on the flexible substrate (300), and
   the radiating element(s) comprise either:

   (i) a plurality of conducting patches (302) arranged in conjunction with the grounded conductor (314) to convey alternating current (AC) to an antenna to deliver said microwave radiation, **characterised in that** each conducting patch (302) has a largest length or width dimension that is a half wavelength of the microwave radiation conveyed by the feed structure when loaded by the tissue to be treated; or **characterised in that** the radiating element(s) comprise :
   (ii) a plurality of non-conducting slots formed in one or more conducting strips (506), each conducting strip (506) being arranged in conjunction with the grounded conductor (314) to convey alternating current (AC) to an antenna to deliver said microwave radiation,

   wherein, in the treatment configuration, the radiating element(s) are arranged to deliver outwardly a substantially uniform radiation field to transfer energy primarily by radiation to the area of oesophageal wall tissue to be treated.

2. A probe according to claim 1, wherein the feed structure (224) includes an impedance transformer (127, 504) having a length of $\dfrac{(2n-1)\lambda}{4}$, where $n$ is an integer and λ is the wavelength of the microwave radiation in the feed structure.

3. A probe according to claim 1 or 2 adapted for use with microwave radiation having a frequency from 5 to 60 GHz.

**4.** A probe according to any preceding claim, wherein the plurality of conducting patches (302) are either connected in series along a transmission line (304) or connected in parallel by transmission lines (308, 310).

**5.** A probe according to claim 1, wherein the feed structure (224) is arranged to cause adjacent ones of the plurality of conducting patches (302) to emit fields that are orthogonal to each other.

**6.** A probe according to any preceding claim, wherein the flexible substrate (300) includes a tubular section mounted on a carrier rod and having axial slits (404) in its surface, which slits (404) define substrate strips that are movable radially outward when the ends of the tubular section are axially moved towards each other, and wherein the radiating element(s) are on the tubular section such that, in use in the treatment configuration, the radiating element(s) are in close proximity or contact with the area of oesophageal wall tissue to be treated.

**7.** A probe according to claim 6, wherein the deployment means (324) includes an axially movable control portion arranged to move the ends of the tubular section (300) relative to each other.

**8.** A probe according to claim 7, wherein the deployment means (324) includes:

driving means mounted on the carrier rod; and
a sleeve axially movably mounted on the carrier rod, the sleeve being movable along the rod by the driving means to cause radial expansion of the tubular array,
wherein the driving means includes a electric current carrying coil around the rod, and the sleeve is arranged to act as a flux multiplier between the coil and the rod to cause it to move axially relative to the rod when current passes through the coil.

**9.** A probe according to any one of claims 1 to 5, wherein the flexible substrate includes one or more paddles (802) movable between an aligned position suitable for insertion into the oesophagus and a transverse spread position in which the radiating elements are in position suitable for treatment.

**10.** A probe according to any one of claims 1 to 5, wherein the flexible substrate includes an inflatable surgical balloon (700) which is radially expandable from the access configuration to the treatment configuration.

**11.** Apparatus for ablating oesophageal wall tissue, the apparatus including:

a source of microwave radiation (100) having a stable output frequency;
a probe (126) according to any preceding claim connected to the source of microwave radiation (100); and
a controller (114) arranged to control the amount of energy delivered by the microwave radiation to the tissue to be treated.

**12.** Apparatus according to claim 11 including a modulator (104) and an attenuator (106) connected between the source of microwave radiation (100) and the probe (126), wherein the controller (114) is arranged to control the modulator (104) and attenuator (106).

**13.** Apparatus according to claim 12 including an amplifier (108) connected between the source of microwave radiation (100) and the probe (126), the amplifier (108) being controllable by the controller (114) to amplify a signal output by the source of microwave radiation (100) to a power level suitable for treating an area of oesophageal wall tissue with microwave radiation.

**14.** Apparatus according to claim 13 including a forward power coupler (110) and a reverse power coupler (112) located between the amplifier (108) and probe (126) to detect a portion of power delivered to and reflected from the oesophageal wall tissue respectively, wherein the controller (114) is arranged to receive signals from the forward power coupler (110) and a reverse power coupler (112) indicative of the detected portions of delivered and reflected power, and to control the modulator (104) and attenuator (106) based on the received signals.

**Patentansprüche**

1. Sonde (226) mit einer Eintrittskonfiguration, in der sie in einen Ösophagus einführbar ist, und einer Behandlungskonfiguration, in der sie zur Behandlung eines Bereichs von Ösophagus-Wandgewebe mit Mikrowellenstrahlung betreibbar ist, wobei die Sonde Folgendes aufweist:

   ein flexibles Substrat (300);
   ein oder mehrere Strahlungselemente auf dem flexiblen Substrat;
   eine Speisungsstruktur (224), die ausgebildet ist, um das/die Strahlungselement(e) mit Energie zu versorgen, sodass das/die Strahlungselement(e) Mikrowellenstrahlung aussendet/n; und
   Einsatzmittel (326), die ausgebildet sind, um die Sonde von der Eintrittskonfiguration in die Behandlungskonfiguration zu versetzen,
   wobei die Speisungsstruktur (224) einen Schutzleiter (314) auf dem flexiblen Substrat (300) umfasst und das/die Strahlungselement(e) entweder

   (i) eine Vielzahl von leitenden Bereichen (302) umfasst/en, die in Zusammenwirkung mit dem Schutzleiter (314) ausgebildet sind, um Wechselstrom (AC) zu einer Antenne zu leiten, um die Mikrowellenstrahlung einzuspeisen, **dadurch gekennzeichnet, dass** jeder leitende Bereich (302) eine maximale Längen- oder Breitenabmessung aufweist, die gleich der halben Wellenlänge der Mikrowellenstrahlung ist, die von der Speisungsstruktur zugeführt wird, wenn sie mit dem zu behandelnden Gewebe beladen ist; oder **dadurch gekennzeichnet, dass** das/die Strahlungselement(e)
   (ii) eine Vielzahl von nichtleitenden Schlitzen umfasst/en, die in einem oder mehreren leitenden Streifen (506) ausgebildet sind, wobei jeder leitende Streifen (506) in Zusammenwirkung mit dem Schutzleiter (314) angeordnet ist, um Wechselstrom (AC) zu einer Antenne zu leiten, um die Mikrowellenstrahlung einzuspeisen,

   wobei das/die Strahlungselement(e) in der Behandlungskonfiguration so ausgebildet ist/sind, dass es/sie ein im Wesentlichen gleichförmiges Strahlungsfeld nach außen hin abgibt/abgeben, um Energie primär mittels Strahlung zum zu behandelnden Bereich des Ösophagus-Wandgewebes zu übertragen.

2. Sonde nach Anspruch 1, worin die Speisungsstruktur (224) einen Impedanzwandler (127, 504) mit einer Länge von $\dfrac{(2n-l)\lambda}{4}$ umfasst, worin n eine ganze Zahl ist und λ die Wellenlänge der Mikrowellenstrahlung in der Speisungsstruktur ist.

3. Sonde nach Anspruch 1 oder 2, die zur Verwendung mit Mikrowellenstrahlung mit einer Frequenz von 5 bis 60 GHz ausgebildet ist.

4. Sonde nach einem der vorangegangenen Ansprüche, worin die Vielzahl von leitenden Bereichen (302) entweder entlang einer Übertragungsleitung (304) in Reihe geschaltet ist oder durch Übertragungsleitungen (308, 310) parallel verbunden ist.

5. Sonde nach Anspruch 1, worin die Speisungsstruktur (224) so ausgebildet ist, dass benachbarte von der Vielzahl von leitenden Bereichen (302) Felder emittieren, die orthogonal zueinander sind.

6. Sonde nach einem der vorangegangenen Ansprüche, worin das flexible Substrat (300) einen röhrenförmigen Abschnitt umfasst, der auf einem Trägerstab befestigt ist und axiale Schlitze (404) auf seiner Oberfläche aufweist, wobei die Schlitze (404) Substratstreifen definieren, die radial nach außen bewegbar sind, wenn die Enden des röhrenförmigen Abschnitts axial zueinander bewegt werden, und worin das/die Strahlungselement(e) sich so auf dem röhrenförmigen Abschnitt befindet/n, dass das/die Strahlungselement(e) bei Verwendung in der Behandlungskonfiguration nahe beim zu behandelnden Bereich des Ösophagus-Wandgewebes liegt/en oder mit diesem in Kontakt ist/sind.

7. Sonde nach Anspruch 6, worin das Einsatzmittel (324) einen axial bewegbaren Kontrollabschnitt umfasst, der ausgebildet ist, um die Enden des röhrenförmigen Abschnitts (300) in Bezug zueinander zu bewegen.

8. Sonde nach Anspruch 7, worin das Einsatzmittel (324) Folgendes umfasst:

ein Antriebsmittel, das auf dem Trägerstab befestigt ist; und
eine Hülse, die axial bewegbar auf dem Trägerstab befestigt ist, wobei die Hülse durch das Antriebsmittel entlang des Stabs bewegbar ist, um eine radiale Ausdehnung der röhrenförmigen Anordnung zu veranlassen, worin das Antriebsmittel eine elektrischen Strom führende Spule um den Stab umfasst und die Hülse angeordnet ist, um als Flussvervielfacher zwischen der Spule und dem Stab zu fungieren, sodass sie sich axial in Bezug zum Stab bewegt, wenn Strom durch die Spule fließt.

9. Sonde nach einem der Ansprüche 1 bis 5, worin das flexible Substrat einen oder mehrere Flügel (802) umfasst, die zwischen einer achsenfluchtenden Stellung, die zur Einführung in den Ösophagus geeignet ist, und einer transversal ausgebreiteten Stellung, in der sich die Strahlungselemente in einer zur Behandlung geeigneten Stellung befinden, bewegbar sind.

10. Sonde nach einem der Ansprüche 1 bis 5, worin das flexible Substrat einen aufblasbaren chirurgischen Ballon (700) umfasst, der aus der Eintrittskonfiguration in die Behandlungskonfiguration radial ausdehnbar ist.

11. Vorrichtung zur Abtragung von Ösophagus-Wandgewebe, wobei die Vorrichtung Folgendes umfasst:

eine Quelle von Mikrowellenstrahlung (100) mit einer stabilen Ausgangsfrequenz;
eine Sonde (126) nach einem der vorangegangenen Ansprüche, die mit der Quelle von Mikrowellenstrahlung (100) verbunden ist; und
eine Steuerung (114), die ausgebildet ist, um Menge an Energie zu steuern, die von der Mikrowellenstrahlung an das zu behandelnde Gewebe abgegeben wird.

12. Vorrichtung nach Anspruch 11, die einen Modulator (104) und einen Abschwächer (106) umfasst, die zwischen der Quelle von Mikrowellenstrahlung (100) und der Sonde (126) angeschlossen sind, worin die Steuerung (114) ausgebildet ist, um den Modulator (104) und den Abschwächer (106) zu steuern.

13. Vorrichtung nach Anspruch 12, die einen Verstärker (108) umfasst, der zwischen der Quelle von Mikrowellenstrahlung (100) und der Sonde (126) angeschlossen ist, wobei der Verstärker (108) durch die Steuerung (114) steuerbar ist, um eine Signalausgabe von der Quelle von Mikrowellenstrahlung (100) auf einen Leistungspegel zu verstärken, der zur Behandlung eines Bereichs von Ösophagus-Wandgewebe mit Mikrowellenstrahlung geeignet ist.

14. Vorrichtung nach Anspruch 13, die einen Vorwärtsleistungskoppler (110) und einen Rückwärtsleistungskoppler (112) umfasst, die zwischen dem Verstärker (108) und der Sonde (126) angeordnet ist, um einen Teil der Leistung zu detektieren, die zum Ösophagus-Wandgewebe zugeführt bzw. von diesem reflektiert wird, worin die Steuerung (114) ausgebildet ist, um Signale von dem Vorwärtsleistungskoppler (110) und dem Rückwärtsleistungskoppler (112) zu empfangen, welche den detektierten Teil an zugeführter und reflektierter Leistung anzeigen, und um den Modulator (104) und Abschwächer (106) basierend auf den empfangenden Signalen zu steuern.

**Revendications**

1. Sonde (226) ayant une configuration d'accès dans laquelle elle peut être insérée dans l'oesophage, et une configuration de traitement dans laquelle elle peut être utilisée pour traiter une zone de tissu de paroi oesophagienne par rayonnement micro-ondes, la sonde comprenant :

un substrat flexible (300) ;
un ou plusieurs éléments rayonnants sur le substrat flexible ;
une structure d'alimentation (224) agencée pour exciter le ou les éléments rayonnants, amenant ainsi le ou les éléments rayonnants à émettre un rayonnement micro-ondes ; et
des moyens de déploiement (326) agencés pour faire passer la sonde de sa configuration d'accès à sa configuration de traitement,
la structure d'alimentation (224) comprend un conducteur relié à la terre (314) sur le substrat flexible (300), et le ou les éléments rayonnants comprennent :

(i) une pluralité de plaques conductrices (302) agencées en conjonction avec le conducteur relié à la terre (314) pour amener un courant alternatif (AC) à une antenne afin de fournir ledit rayonnement micro-ondes, **caractérisée en ce que** chaque plaque conductrice (302) présente la plus grande dimension en longueur ou largeur d'une demi-longueur d'onde du rayonnement micro-ondes amené par la structure d'alimentation lorsqu'elle est chargée avec le tissu à traiter ; ou

**caractérisée en ce que** le ou les éléments rayonnants comprennent :

(ii) une pluralité de fentes non conductrices formées dans une ou plusieurs bandes conductrices (506), chaque bande conductrice (506) étant agencée en conjonction avec le conducteur relié à la terre (314) pour amener un courant alternatif (AC) à une antenne afin de fournir ledit rayonnement micro-ondes,

dans laquelle, dans la configuration de traitement, le ou les éléments rayonnants sont agencés pour fournir vers l'extérieur un champ de rayonnement substantiellement uniforme afin de transférer de l'énergie principalement par rayonnement à la zone de tissu de paroi oesophagienne à traiter.

2. Sonde selon la revendication 1, dans laquelle la structure d'alimentation (224) comprend un convertisseur d'impédance (127, 504) de longueur $\frac{(2n-1)\lambda}{4}$ où n est un entier et λ est la longueur d'onde du rayonnement micro-ondes dans la structure d'alimentation.

3. Sonde selon la revendication 1 ou 2, adaptée pour une utilisation avec un rayonnement micro-ondes ayant une fréquence de 5 à 60 GHz.

4. Sonde selon l'une quelconque des revendications précédentes, dans laquelle la pluralité de plaques conductrices (302) est soit connectée en série le long d'une ligne de transmission (304), soit connectée en parallèle par des lignes de transmission (308, 310).

5. Sonde selon la revendication 1, dans laquelle la structure d'alimentation (224) est agencée pour amener des plaques adjacentes de la pluralité de plaques conductrices (302) à émettre des champs qui sont orthogonaux les uns par rapport aux autres.

6. Sonde selon l'une quelconque des revendications précédentes, dans laquelle le substrat flexible (300) comprend une section tubulaire montée sur une tige de support et ayant des fentes axiales (404) dans sa surface, lesquelles fentes (404) définissent des bandes de substrat qui sont mobiles radialement vers l'extérieur lorsque les extrémités de la section tubulaire sont rapprochées axialement l'une de l'autre, et dans laquelle le ou les éléments rayonnants se trouvent sur la section tubulaire de telle sorte qu'en cours d'utilisation dans la configuration de traitement, le ou les éléments rayonnants se trouvent à proximité immédiate ou en contact avec la zone de tissu de paroi oesophagienne à traiter.

7. Sonde selon la revendication 6, dans laquelle les moyens de déploiement (324) comprennent une portion de commande axialement mobile agencée pour déplacer les extrémités de la section tubulaire (300) l'une par rapport à l'autre.

8. Sonde selon la revendication 7, dans laquelle le moyen de déploiement (324) comprend :

des moyens d'entraînement montés sur la tige de support ; et
une gaine montée de manière axialement mobile le long de la tige de support, la gaine étant mobile le long de la tige par le moyen d'entraînement afin de provoquer une dilatation radiale de l'ensemble tubulaire, dans laquelle le moyen d'entraînement comprend une bobine porteuse de courant électrique autour de la tige, et la gaine est agencée pour agir comme un multiplicateur de flux entre la bobine et la tige afin de l'amener à se déplacer axialement par rapport à la tige lorsque le courant passe par la bobine.

9. Sonde selon l'une quelconque des revendications 1 à 5, dans laquelle le substrat flexible comprend une ou plusieurs spatules (802) mobiles entre une position alignée adaptée pour une insertion dans l'oesophage et une position étalée transversale dans laquelle les éléments rayonnants se trouvent dans une position adéquate pour le traitement.

10. Sonde selon l'une quelconque des revendications 1 à 5, dans laquelle le substrat flexible comprend un ballonnet (700) chirurgical gonflable qui peut être dilaté radialement de la configuration d'accès à la configuration de traitement.

**11.** Appareil pour l'ablation de tissu de paroi oesophagienne, l'appareil comprenant :

une source de rayonnement micro-ondes (100) ayant une fréquence de sortie stable ;
une sonde (126) selon l'une quelconque des revendications précédentes, connectée à la source de rayonnement micro-ondes (100) ; et
un contrôleur (114) agencé pour commander la quantité d'énergie fournie par le rayonnement micro-ondes au tissu à traiter.

**12.** Appareil selon la revendication 11, comprenant un modulateur (104) et un atténuateur (106) connectés entre la source de rayonnement micro-ondes (100) et la sonde (126), dans lequel le contrôleur (114) est agencé pour commander le modulateur (104) et l'atténuateur (106).

**13.** Appareil selon la revendication 12, comprenant un amplificateur (108) connecté entre la source de rayonnement micro-ondes (100) et la sonde (126), l'amplificateur (108) pouvant être commandé par le contrôleur (114) pour amplifier un signal délivré par la source de rayonnement micro-ondes (100) à un niveau d'énergie adéquat pour traiter une zone de tissu de paroi oesophagienne par rayonnement micro-ondes.

**14.** Appareil selon la revendication 13, comprenant un coupleur d'énergie direct (110) et un coupleur d'énergie inverse (112) situés entre l'amplificateur (108) et la sonde (126) afin de détecter une partie d'énergie fournie à et réfléchie par le tissu de paroi oesophagienne, respectivement, dans lequel le contrôleur (114) est agencé pour recevoir des signaux du coupleur d'énergie direct (110) et d'un coupleur d'énergie inverse (112) indiquant les parties détectées de l'énergie fournie et réfléchie, et pour commander le modulateur (104) et l'atténuateur (106) sur la base des signaux reçus.

FIG. 1

FIG. 2

FIG. 3

FIG. 3A

FIG. 3B

FIG. 4

FIG. 5

FIG. 6

FIG. 7

FIG. 9

FIG. 10

FIG. 8

EP 2 068 741 B1

FIG. 11A

EP 2 068 741 B1

FIG. 11B

EP 2 068 741 B1

502  504  506  508

**FIG. 12**

EP 2 068 741 B1

FIG. 13

302  300

320

324  322  330

FIG. 14

328

324          326

328

FIG. 15

300

326

300

324

324

326

FIG. 17A

FIG. 17B

324  332  334

328

336

338

328

I  8

340

FIG. 16

EP 2 068 741 B1

FIG. 18

FIG. 19

FIG. 20

FIG. 21

510

512

514

$\dfrac{(2n-1)\lambda}{4}$

526

516

508

524

518

W

522

520

**FIG. 22**

0.3mm

0.3    0.3    0.3    0.3    0.3    0.3

1    2    3    4    5    6    7    8

w/2    0.867mm    w/2

0.433mm

**FIG. 23**

FIG. 25

FIG. 24

FIG. 26

$a = d_2 - t_2$      $d_1 = 0.6mm$
$b = d_1 + t_1$      $d_2 = 2.7mm$
                  $t_1 = 10\mu m$
                  $t_2 = 10\mu m$

FIG. 27

EP 2 068 741 B1

IN →

706

704

702

700

FIG. 28A

FIG. 28B

46

$$\frac{(2n-1)\lambda}{4}$$

$$Z_0 = \sqrt{50 \times 5} = \underline{15.8\ \Omega}$$

$$Z_S = Z_L = 5\Omega$$

808  $Z_0 = 50\Omega$

806

810

$Z_0 = 50\Omega$

$$\frac{(2n-1)\lambda}{2}$$

804

$$Z_L = \frac{20}{4} = 5\Omega$$

802

802

$Z_0 = 20\Omega$

802

802

$Z_0 = 20\Omega$

$Z_0 = 20\Omega$

$Z_0 = 20\Omega$

$Z_0 = 20\Omega$

FIG. 29

FIG. 30

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20030009165 A
- US 20040002747 A
- US 6024743 A
- US 20050096647 A
- WO 2005112812 A
- US 20030069620 A
- IT 19972002

### Non-patent literature cited in the description

- **GANG et al.** Microstrip Antenna Design Handbook. Artech House, 2001